(19) Europäisches Patentamt
European Patent Office
Office européen des brevets

(11) Publication number: 0 328 106 A2

# (12) EUROPEAN PATENT APPLICATION

(21) Application number: 89102245.1

(22) Date of filing: 09.02.89

(51) Int. Cl.[4]: G01N 33/58 , G01N 33/543 , G01N 33/52

(30) Priority: 09.02.88 JP 29632/88
09.02.88 JP 29633/88

(43) Date of publication of application:
16.08.89 Bulletin 89/33

(84) Designated Contracting States:
DE GB

(71) Applicant: KONICA CORPORATION
No. 26-2, Nishishinjuku 1-chome Shinjuku-ku
Tokyo 160(JP)

(72) Inventor: Onishi, Akira
c/o Konica Corporation 1 Sakuramachi
Hino-shi Tokyo(JP)
Inventor: Kawakatsu, Satoshi
c/o Konica Corporation 1 Sakuramachi
Hino-shi Tokyo(JP)
Inventor: Ito, Tsukasa
c/o Konica Corporation 1 Sakuramachi
Hino-shi Tokyo(JP)
Inventor: Takahashi, Takenori
c/o Konica Corporation 1 Sakuramachi
Hino-shi Tokyo(JP)
Inventor: Fukaya, Michie
c/o Konica Corporation 1 Sakuramachi
Hino-shi Tokyo(JP)

(74) Representative: Henkel, Feiler, Hänzel & Partner
Möhlstrasse 37
D-8000 München 80(DE)

## (54) Method of assaying substance and immunoassay element.

(57) Disclosed is a method of assaying a target substance in a fluid sample. In this method, a) the target substance, b) a substance which specifically binds to the target substance, to which a biological active substance which does not bind to the target substance is attached, or to which a substance which specifically binds to a biological active substance which does not bind to the target substance is attached; c) a labelled substance which is the target substance or an analogue thereof labelled with β-D-galactosidase, or which is a substance which specifically binds to the target substance, labelled with β-D-galactosidase, d) a substance which specifically binds to the biological active substance and which does not bind to the target substance, or the biological active substance, which is fixed to a carrier, which carrier exists in a porous reaction layer of an assay element, and e) a substance which specifically binds to β-D-galactosidase and which changes a signal originated from β-D-galactosidase, which is fixed to said carrier or another carrier which exists in a porous reaction layer of an assay element are reacted and the change of the signal from β-D-galactosidase is measured.

EP 0 328 106 A2

# METHOD OF ASSAYING SUBSTANCE AND IMMUNOASSAY ELEMENT

## SPECIFICATION

## BACKGROUND OF THE INVENTION

### I. Field of the Invention

This invention relates to a method of assaying a target substance of which existance is to be detected or of which amount is to be determined. This invention also relates to an immunoassay element for conducting the method of the present invention.

### II. Description of the Related Art

A number of assay for detecting a substance contained in a biological fluid in an very small amount have been developed. Among these, the most sensitive assay is the immunoassay.

Immunoassay was first invented by Berson and Yallow in 1958. They succeeded in quantifying insulin in blood using bovine insulin labelled with radioactive iodine and anti-insulin antibody. Thereafter, radioimmunoassay has been widely used.

Various markers other than the radioactive markers have been developed. The markers other than the radioactive markers include enzymes, substrates of enzymes, coenzymes, enzyme inhibitors, bacteriophages, circulation reactant, complexes of metals or organic metals, organic prosthetic groups, chemiluminescent reactant and fluorescent molecules.

One of the important technical problems in immunoassay is so called B/F separation by which a bound substance and free substance are separated.

To solve the problems in the conventional immunoassay methods, various methods have been proposed (for example, Japanese patent disclosure (Kokai) Nos. 38619/78, 79024/78, 90859/80, 67680/82, 200862/82, 18167/83, 77356/84 and 170768/84).

However, these methods have drawbacks in, for example, that the B/F separation is incomplete, or noise is so large that the obtained signals are not reliable.

On the other hand, in the field of wet chemistry assay, immunoassay methods based on the competition reaction employing a solid phase reactant have been developed (for example, Japanese Patent Disclosure (Kokai) Nos. 209994/83 and 202064/84). However, in these methods, since a competivite reaction is conducted among the two components fixed to a solid carrier and the components in the solution, the components likely remain in the solution without conducting the intended reaction. Further, since the entire enzyme activity is measured without separating the two solid phase reactants, the sensitivity, precision and reproducibility of the methods are not satisfactory due to the background or noise.

On the other hand, in the field of dry chemistry assay, the methods in which a second antibody is used have been developed (for example, Japanese Patent Disclosure (Kokai) Nos. 82776/82 and 82767/82).

However, these methods have drawbacks in that the operation is troublesome and a technique for spreading the sample with high reproducibility is required. Japanese Patent Disclosure (Kokai) No. 34155/84 discloses a method in which a sheet containing non-bound component is held. However, even with this method, if the measurement is conducted while intimately contacting the sheet for reaction and the non-bound component-containing sheet, the above-described problems are brought about. Further, to separate the sheets at the time of measurement is troublesome and makes it difficult to automate the operation.

## SUMMARY OF THE INVENTION

Accordingly, the object of the present invention is to provide a method of assaying a target substance in a sample fluid, in which high degree of B/F separation is conducted, which has low background and

noise, and so which has high sensitivity, precision and reproducibility.

The present inventors have intensively studied to find that by employing β-D-galactosidase as a marker, an assay method with excellent sensitivity, precision and reproducibility can be provided, to reach the present invention.

That is, the present invention provides a method of assaying a target substance in a fluid sample utilizing a specific binding reaction between the target substance and a substance which specifically binds to the target substance, comprising the steps of reacting a) the target substance, b) the substance which specifically binds to the target substance, to which a biological active substance which does not bind to the target substance is attached, or to which a substance which specifically binds to a biological active substance which does not bind to the target substance is attached, c) a labelled substance which is the target substance or an analogue thereof labelled with β-D-galactosidase, or which is a substance which specifically binds to the target substance, labelled with β-D-galactosidase, d) a substance which specifically binds to the biological active substance, or the biological active substance, which is fixed to a carrier, which carrier exists in a porous reaction layer of an assay element, and e) a substance which specifically binds to β-D-galactosidase and which changes a signal originated from β-D-galactosidase, which is fixed to said carrier or another carrier which exists in a porous reaction layer of an assay element, and measuring the change of the signal from β-D-galactosidase, thereby detecting the existance of the target substance or determining the amount of the target substance in the fluid sample.

The present invention also provides an immunoassay element comprising a biological active substance which does not binds to a target substance in a fluid sample, or a substance which specifically binds to a biological active substance which does not binds to a target substance in a fluid sample, which is fixed to a carrier contained in a porous reaction layer of the immunoassay element; and a substance which specifically binds to β-D-galactosidase and which changes a signal originated from β-D-galactosidase, which is fixed to said carrier or another carrier existing in the porous reaction layer of the immunoassay element.

The present invention further provides a method of assaying a target substance in a fluid sample utilizing a specific binding reaction between the target substance and a substance which specifically binds to the target substance, comprising the steps of reacting a) the target substance, b) a labelled substance which is the target substance or an analogue thereof labelled with β-D-galactosidase, or a labelled substance which is a substance which specifically binds to the target substance, labelled with β-D-galactosidase, c) the substance which specifically binds to the target substance, which is fixed to a carrier contained in a porous reaction layer of an immunoassay element, and d) a substance which specifically binds β-D-galactosidase and which changes a signal originated from β-D-galactosidase, which is fixed to said carrier or another carrier which exists in a porous reaction layer of an assay element; and mesuring the change of the signal from β-D-galactosidase, thereby detecting the existence of the target substance or determining the amount of the target substance in the fluid sample.

The present invention still further provides an immunoassay element comprising a substance which specifically binds to a target substance in a fluid sample, which is fixed to a carrier existing in a porous reaction layer of the immunoassay element; and a substance which specifically binds to β-D-galactosidase and which changes a signal originated from β-D-galactosidase, which is fixed to said carrier or another carrier existing in a porous reaction layer of the immunoassay element.

By the method of the present invention, as will become more apparent from the description below, B/F separation can be attained with higher level than in the conventional methods, and so the background and noise are low. Therefore, the sensitivity, precision and reproducibility of the present invention are excellent.

## BRIEF DESCRIPTION OF THE DRAWINGS

Figs. 1, 4 - 9 and 12 are schematical cross-sectional views showing the examples of the layered structure of the embodiments of the immunoassay element of the present invention;

Fig. 2 is a schematic enlarged view of the porous reaction layer of an embodiment of the immunoassay element of the present invention;

Figs. 3 and 3′ schematically shows the principle of the method of the present invention;

Fig. 10 is a schematical cross sectional view of an embodiment of the immunoassay element of the present invention;

Fig. 11 is a schematical perspective view of the embodiment of the immunoassay element of the present invention shown in Fig. 10;

Figs. 13 - 16 are calibration curves obtained in quantifying human IgG in samples, according to the method of the present invention;

Figs. 17 - 40 show the result of the measurement human IgG in samples, obtained by employing the method of the present invention; and

Figs. 41 - 44 are calibration curves obtained in quantifying human IgG in samples, according to the method of the present invention.

## DETAILED DESCRIPTION OF THE PREFERRED EMBODIMENTS

The fluid sample which may be subjected to the assaying method of the present invention may be any solution or colloidal solution. Typically, the fluid sample may be a biological fluid such as blood, plasma, serum, cerebrospinal fluid, saliva, amniotic fluid, milk, urine, sweat, broth and the like.

The target substance contained in the fluid sample is the substance which is to be assayed, i.e., the substance of which existence is to be detected or of which amount is to be determined. Examples of the target substance may include polypeptides, proteins, conjugated proteins, carbohydrates, lipids, complex lipids, nucleic acids, hormones or hormone-like substances, vitamins, drugs and metabolites thereof, antibiotics and agricultural chemicals. It is necessary that it is possible to provide a substance such as an antibody which specifically reacts or binds the target substance. Non-limiting specific examples of the target substance will now be enumerated.

[Proteins and Protein Complexes]

Prealbumin, albumin, $\alpha_1$-acidic glycoprotein, $\alpha_1$-antitrypsin, $\alpha_1$-antichymotrypsin, $\alpha_1$-lipoprotein, thyroxine-bound globulin, ceruloplasmin, Zn-$\alpha_2$-glycoprotein, Gc-globulin, inter-$\alpha$-trypsin inhibitor, $\alpha_1$-macroglobulin, $\alpha_2$-HS-glycoprotein, $\alpha_2$-macroglobulin, haptoglobin, $\alpha_2$-lipoprotein, hemopexin, transferin, $\beta$-lipoprotein, $\beta_2$-glycoprotein, $\beta_2$-macroglobulin, C-reactive protein, myoglobin, erythropoietin, immunoglobulin (IgG, IgM, IgA, IgD, IgE), complement component ($C_1q$, $C_1r$, $C_1s$, $C_2$, $C_3$, $C_4$, $C_5$, $C_6$, $C_7$, $C_8$, $C_9$ and the like), fibrinogen, hemoglobin, glycohemoglobin, blood coagulating factor, HBs antigen, HBs antibody, enzymes (e.g., acid phosphatase, alkaline phosphatase, alkaline phosphatase isoenzyme, $\alpha$-amylase, amylase isoenzyme, aldolase, choline esterase, creatin phosphokinase, creatin phosphokinase isoenzyme, transamynase (GOT, GPT), lactate dehydrogenase, lactate dehydrogenase isoenzyme, $\gamma$-GTP, lipase monoamineoxidase, leucineaminopeptidase, glucose-6-phosphate dehydrogenase, and the like)

[Hormones and Hormone-like Substances]

Follicle stimulating hormone (FSH), luteotropic hormone (LH), growth hormone (GH), thyroid stimulating hormone (TSH), adrenocorticotropic hormone (AcTH), melanin stimulating hormone (MHS), vasopressin, oxytocin, insulin, glucagon, angiotensin I, II, prolactin, secretin, dopamine, serotonin, somatostatin, thyroxine ($T^4$), triiodothyronine ($T^3$), gastrin, cortisol, aldosterone, catecholamine, estrogen, progesteron, testosterone, placental gonadotropin, placental lactogen, pituitary hormone releasing factors (TRH, FSH-RH, CRH, LH-RH and the like).

[Vitamins]

Biotin, thiamine, vitamin A, vitamin $B^2$, vitamin $B^6$, vitamin $B^{12}$, vitamin C, vitamin D, vitamin E, vitamin K and folic acid.

[Tumor Markers}

$\alpha$-fetoprotein, carcinoembryonic antigen, ferritin, polyamine, pancreas carcinoembryonic antigen, basic fetoprotein, M-protein, prostate gland acid phosphatase, carbohydrate antigen (CA19-9, CA125, etc.), ganglioside.

[Drugs and Metabolities Thereof]

Benzoyl ecgonine, cocaine, codeine, dextromethorphan, heroin, lysergic acid, morphine, quinidine, quinine, amycacin, gentamycin, kanamycin, neomycin, tobramycin, actinomycetin, karomycin, chloramphenicol, chloromycetin, chlorotetracycline, erythromycin, oxytetracycline, penicillin, cephalosporin, polymyxin B, terramycin, tetracycline, streptomycin, diphenylhydantoin, ethosuximide, phenobarbital, primidone, secobarbital, acetaminophenone, amitriptyline, carbamazepin, digoxin, disopyramide, lidocaine, methotrexate, N-acetylprocainamide, phenytoin, procainamide, propranolol, theophyllin, canabinol, tetrahydrocanabinol, choline inhibitory drug, antihistaminic agent, atropine, butyrophenone, caffeine, chloropromazine, barbiturate, amphetamine, catecholamine, epinephrine, griseofulvin, imipramine, L-DOPA, meperidine, meprobamate, methadone, narceine, nortriptyline, oxazepam, papaverine, prostaglandin and valproic acid, as well as the metabilites thereof.

[Microorganism surface marker]

Halogenated biphenyl, phosphate esters, thiophosphates and metabolities thereof.

[Agricultural Chemicals}

Halogenated biphenyl, phosphate esters and thiophosphates, as well as the metabolites thereof.

[others]

Blood group substances, cardiolipin and allergen.

In the method of the present invention, the substance which specifically binds to the target substance (hereinafter referred to as "target-binding substance" is employed. The target-binding substance may be, for example, an antibody, antigen, lectin or protein A depending on the target substance. It is preferred that the reaction between the target substance and the target-binding substance be an antigen-antibody reaction. The antibody which may be used as the target-binding substance may be obtained by a well-known conventional method. That is, the antibody may be produced by immunizing an animal with the target substance. In cases where the target substance has no immunogenecity, a conjugate of the target substance and a carrier protein such as albumin may be used for the immunization. Such a conjugate is well-known in the art. The antiserum or ascites collected from the immunized animal may be used as it is. Alternatively, the antibody may be used after purification by a well-known method such as sodium sulfate sedimentation method, ammonium sulfate precipitation method, gel filtration method by using, for example, Sephadex gel (commercially available from Pharmacia), ion exchange cellulose chromatography method and electrophoresis method (the purification method is described in detail in, for example, Shunsuke Uda ed. "Immunochemistry", pp.74-84, published by Nakayama Shoten). Alternatively, a monoclonal antibody may also be employed. The monoclonal antibody specific to the target substance may be prepared by the well-known hybridoma technology. Using a monoclonal antibody is preferred in view of the high specificity. Monoclonal antibody in any class such as IgG, IgM, IgA, IgD and IgE, as well as a polyclonal antibody or antiserum may be used. Futher, Fab, Fab′ and $F(ab')_2$ fragments with antibody activity may also be used. These antibodies or fragments thereof may be used individually or in combination. In cases where the target substance is an antibody, the corresponding antigen may conveniently used as the target-binding substance.

In the method of the present invention, a biological active substance which does not bind to the target substance, or a substance which specifically binds to a biological active substance (BAS for short) is attached to the target-binding substance. It is necessary that the biological active substance should have a substance which specifically binds to the biological active substance (hereinafter referred to as "biological substance-binding substance' or (BSBS) for short). The combination of the biological active substance and the biological substance-binding substance may include the following:

| BAS | BSBS |
|---|---|
| Enzymes: | Substrates (products)<br>Inhibitors<br>Prosthetic groups<br>Allosteric Effectors |
| Antibodies: | Antigens<br>Protein A |
| Lectins: | Polysaccharides<br>Glycoproteins |
| Nucleic Acids: | Complementary Base Sequences<br>Histones<br>Nucleic Acid Polymerase |
| Hormones: | Receptors |
| Biotin (biocytin, desthiobiotin, oxybiotin) | Avidin (Streptoavidin) |

Among these, the preferred combination is antibody-antigen and biotin-avidin. The most preferred is biotin-avidin. The biological active substance or the biological substance-binding substance is chosen so as not to react or interact with the target substance or labelled substance hereinbelow described which is used in the assay method.

In the method of the present invention, a labelled substance is employed. The labelled substânce is the target substance or an analogue thereof which is labelled with β-D-galactosidase, or a substance which specifically binds to the target substance, labelled with β-D-galactosidase. Using β-D-galactosidase as a marker is one of the most characteristic features of the present invention. β-D-galactosidase (EC.3.2.1.23) per se is a well-known enzyme. β-D-galactosidase of any origin can be used. The β-D-galactosidases from animals, plants, or microorganisms which are described in, for example, Bunji Maruo, Nobuo Tamiya eds. "Enzyme Handbook", vol. 7 "Enzymes", pp.617-663, (published by Asakura Shoten, 1982) are preferred. Among these, the most preferred is the β-D-galactosidase from E. coli.

The methods of labelling a substance with an enzyme without losing the enzyme activity is well-known in the art, and the known methods may be employed for labelling a substance with β-D-galactosidase in the present invention. Suċh ȧ method is described, for example, in Eiji Ishikawa, Tadashi Kawai, Kiyoshi Miyai eds. "Enzyme Immunoassay (second edition)" (published by Igakushoin, 1978) or "Clinical Pathology", Extraordinary Vol. 53, "Immunoassay for Clinical Examination, -Technique and Application" Japan Clinical Pathology Association eds., published by clinical pathology publisher, 1983). The concrete and detailed methods are described in Japanese Patent Application No. 280166/86.

In the method of the present invention, a substance which specifically binds to the biological active substance described above, or the biological active substance, which is fixed to a carrier is employed. The carrier exists in a porous reaction layer of an assay element. The porous reaction layer will be hereinbelow described in detail.

In the method of the present invention, a substance which specifically binds to β-D-galactosidase and which changes a signal originated from β-D-galactosidase (hereinafter referred to as "signal-changing substance"), which is fixed to a carrier is employed. The carrier may be the same carrier on which the substance which specifically binds to the biological active substance described above, or the biological active substance is fixed, ot may be another carrier which exists in the porous reaction layer. In view of the better B/F separation, the latter mode is preferred. The substance which specifically binds to β-D-galactosidase and which changes a signal originated from β-D-galactosidase is one which gives influence on the enzyme activity of β-D-galactosidase. Preferred signal-changing substances are inhibitors of β-D-galactosidase, examples thereof may include organic mercury derivatives described in Japanese Patent Disclosure (Kokai) No. 249063/87, more preferably aryl mercury compounds. Also preferred are organic silver compounds, galactostatins, p-aminophenyl-β-D-thiogalactopyranoside and o-mercuryphenyl-β-D-galactoside chloride.

The aryl mercury derivatives which may be employed as the signal-changing substance in the present invention are represented by the general formula of $R(HgX)^n$. In this formula, "R" represents benzene or naphthalene ring which may be substituted. Examples of the substituents may include halogen atoms (F, Cl, Br, I) -CN, $-NO_2$, -SH,

$-OR$, 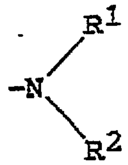 , $-COOR^1$, $-COR^1$, 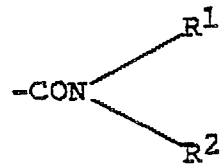 ,

$-OCOR^1$, 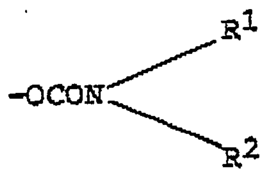 , $-NHCOR^1$, $-NHSOOR^1$

$-SO_3R^1$, $-SO_2R^1$,

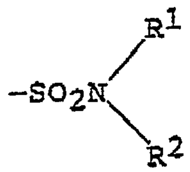

alkyl groups which may be substituted and phenyl group which may be substituted. The "$R^1$ and "$R^2$ in the above formulae represent alkyl groups, phenyl group which may be substituted or hydrogen. The number of the substituents on the benzene ring and the naphthalene ring is not more than 7, and preferably not more than 3. The "X" in the above formula is a monovalent acid group. The "n" in the above formula is an integer of 1 or 2. Specific examples of the aryl mercury derivatives are summarized in the Table 1 below.

Table 1

1 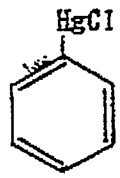

2 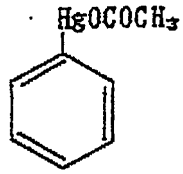

3 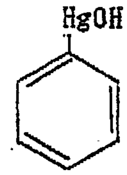

4 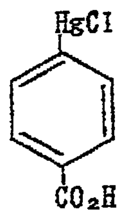

5 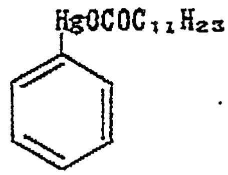

6 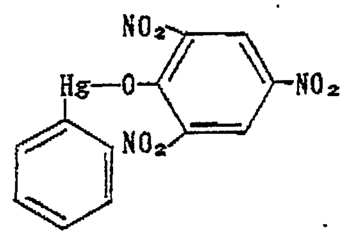

7 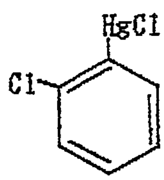

8 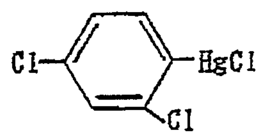

9 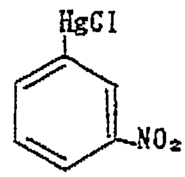

10 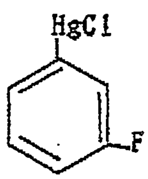

11 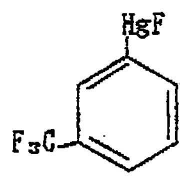

12 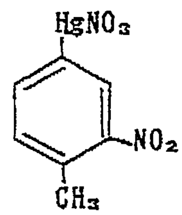

13 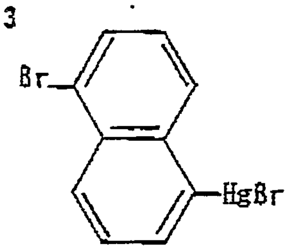

14 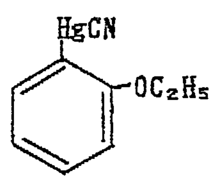

15 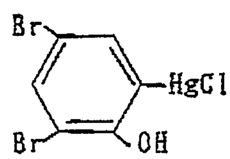

Table 1 (continued)

16 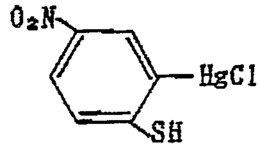

17 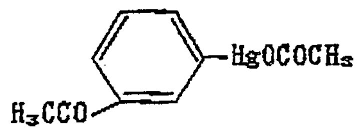

18 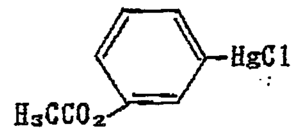

19 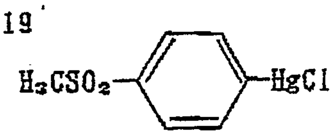

20 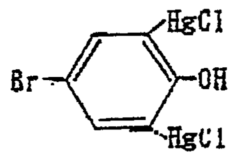

21 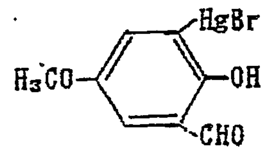

22 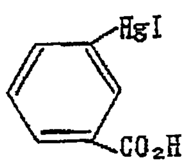

23 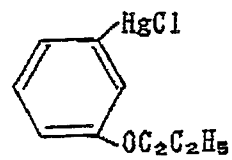

24 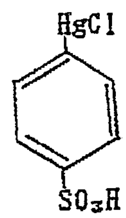

25 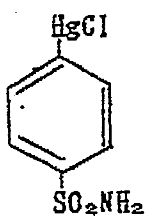

26 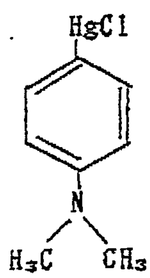

27 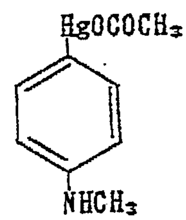

28 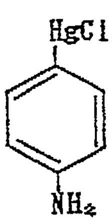

29 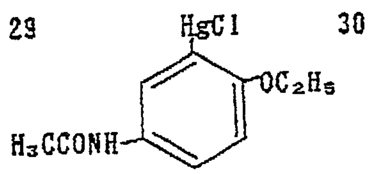

30 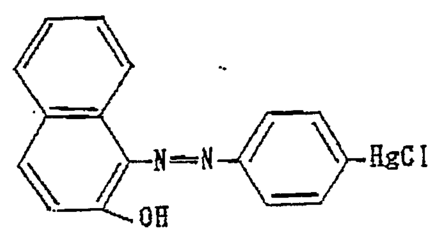

Still other aryl mercury derivatives are described in detail in Beilsteins Handbuch der Organischen Chemie, 4th edition, vol. XIV, second supplement, pp. 1347-1424. The aryl mercury derivatives may be prepared by known methods, which are described in, for example, the reference just mentioned above.

The term "porous reaction layer" means that the porous layer in which the reaction required in the present invention, which is hereinbelow described in detail, can be carried out. For accomplishing this, the porous reaction layer should have a porous structure having communication holes preferably having pore diameter of 1 - 300 µm in part of, or in the entire layer.

As long as this requirement is satisfied, the material for constituting the porous reaction layer is not limited. Preferably, the porous reaction layer is made of particles (i.e., a material in powder form) with a particle size of 1 - 350 µm, and/or a fabric with a mesh size of 40 - 400 mesh. Preferred examples of the material in powder form may include diatomaceous earth, titanium dioxide, barium sulfate, zinc oxide, lead

oxide, microcrystalline cellulose, siliceous sand, glass, silica gel, crosslinked dextrane, crosslinked polyacrylamide, agarose, crosslinked agarose and various synthetic polymer such as polystyrene, as well as the self-binding particles consisting essentially of the compounds having the reactive groups such as oxiranyl group, glycidyl group, aziridinyl group, hydroxy group, amino group, carboxy group, thiol group, isocyanate group and vinyl sulfonyl group, which are described in detail in Japanese Patent Disclosure (Kokai) Nos. 101760/82 and 101761/82. These materials may be employed individually or in combination. The size of the particles constituting the powder may preferably be 1 - 100 µm.

In cases where the porous reaction layer is made of a fabric, examples of the material for forming the porous reaction layer may include pulp, cellulose powder and natural, synthetic and semisynthetic fibers of various plants, animals, minerals and artificial products such as cotton, hemp, silk, wood, chitin, chitosan, cellulose ester, viscose rayon, copper-ammonia rayon, polyamides (e.g., 6-Nylon, 6,6-Nylon and the like), polyesters (e.g., polyethylene terephthalate and the like), polyolefins (polypropyrene, vinylon and the like), glass and asbestos. These materials may be employed individually or in combination. Further, water-absorptive European paper, Japanese paper, filter paper and brush polymer, as well as the woven fabrics, non-woven fabrics and synthetic paper made from one or more of the above-described fibers may also be employed.

In cases where the porous reaction layer is made of powder material such as microcrystalline cellulose or non-self adhering particles, an appropriate adhesive or binder is used to mutually adhering the powder material or the particles. The adhesive which may be employed is described in Japanese Patent Disclosure (Kokai) Nos. 53888/74, 90859/80 and 67860/82. In cases where the particles constituting the powder material have self-adhering property, the reaction layer may be prepared by a known method described in, for example, Japanese Patent Disclosure (Kokai) Nos. 101760/82, 101761/82 and 70163/83.

In cases where the porous reaction layer is made of fibers or fibers-particles mixture, the reaction layer may be prepared by applying a dispersion of fibers or fibers-particles mixture and drying the dispersion. Such a dispersion is known in the art and is described in, for example, Japanese Patent Disclosure (Kokai) Nos. 125847/82 and 197466/82. Dispersion may also be prepared by, as described in Japanese Patent Disclosure (173471/85) using a hydrophilic binder such as gelatin, polyvinylpyrrolidone and polyvinyl alcohol. The content of the hydrophilic binder may be selected from the wide range, and may preferably be 0.1 - 25% by weight, more preferably 1.0 - 20% by weight with respect to the weight of the fibers or the filbers-particles mixture. To prepare such a dispersion, various method may be employed individually or in combination. A useful method is to add a surface active agent to the medium of the dispersion. Preferred surface active agents are non- ionic and ionic surface active agents such as octylphenoxypolyethoxyethanol (commercially available from Rohm and Haas under the trade name of Triton X-100), nonylphenoxypolyglycidol (commercially available from Oline Co., Ltd. under the tradename of Surfactant 10G). The concentration of the surface active agent may be selected from a wide range, and may preferably be 20 -0.005% by weight, more preferably 15 - 0.1% by weight with respect to the weight of the fibers or fibers-particles mixture. Further, ultrasonic treatment, physical mixing and/or agitation treatment may also be employed for the preparation of the dispersion. These methods are more useful if they are combined with the above-mentioned treatment with the surface active agent.

The fibers and/or particles constituting the porous reaction layer may be used as the carrier to which the substance which specifically binds to the biological active substance, biological active substance, or the signal-changing substance is fixed. Fixing of these substances may be attained by various well-known methods. That is, these substances may be fixed to the surface of the porous reaction layer by physical adsorption or by chemical bond directly or indirectly. In fixing these substances, the activity thereof should not be lost. Fixing these substances to a carrier without losing the activities may be conducted, by known methods such as, for example, disclosed in Eiji Ishikawa, Tadashi Kawai, Kiyoshi Miyai eds. "Enzyme Immunoassay (Second Eds.)" published by Igaku Shoin, 1978, or in Ichiro Senhata, Tetuya Tosa and Yuji Matsuo "Experiments and Application, Affinity Chromatography" published by Kodansha, 1976). Further, as long as the specific binding sites of these substances are retained and the substances are not liberated to or soluted in the fluid sample, these substances may be dispersed in the fluid sample in the immobilized state. Further, the method for dispersing couplers in the color photography field (e.g., the method described in Japan Photography Association eds. "Basics of Photography Technology, Silver Salts" published by Corona Co., Ltd., 1978), and the method in which the substances is encapsulated in lipid bilayered membranes may be employed. After immobilizing the target-binding substance, if necessary, to eliminate the non-specific reaction in the immunological reaction, a protein which does not participate the specific reaction in interest may be fixed to the carrier. Representative examples of these proteins may include normal serum proteins of mammals, albumin and gelatin, as well as the decomposed products thereof.

The fixing operation may preliminarily be performed on the particles of fibers and thereafter the porous

reaction layer may be formed. Alternatively, the porous reaction layer may be formed first and then the fixing operation can be performed on the formed reaction layer. In the former case, fibers or particles to which the above-described proteins which do not participate the specific reaction may be added.

The mixing ratio of the two kinds of the carriers may appropriately be selected depending on the amount of the substance fixed and on the binding constant. Further, carriers to which these substances are not bound may be added for adjustment.

The above-mentioned two kinds of carriers may exist in a single layer in the porous reaction layer. Alternatively, the porous reaction layer may comprise more than one sublayers and the two kinds of carriers exist in the separate sublayers.

The mode of the assay element of the present invention is not limited as long as the above-mentioned reaction can be carried out. However, in view of the ease of manufacturing and in view of the ease of operation, those in the form of a film or sheet are preferred.

After the above-mentioned reaction is conducted, the change of the signal originated from β-D-galactosidase is mesured. The signal originated from β-D-galactosidase can be detected by measuring absorbance (colorimetry), fluorescent light or by measuring the generated color by, for example, measuring the reflection density. These methods are known in the art, and are described in, for example, Eiji Ishikawa, Tadashi Kawai and Kiyoshi Miyai eds. "Enzyme Immunoassay (second edition)" (Igaku Shoin, 1978). The rate assay in which the change of the signal with time is measured or end point assay in which the signal at a certain time is measured may be employed. The most preferred method is the colorimetry, because it can be conducted using a relatively simple apparatus and judgement by gross examination can also be conducted. In the colorimetry, ultraviolet light, visible light and near infrared light may be employed. In cases where the fluid sample is blood or urine, to reduce the influence of the sample to the measuring light, green, red or near infrared light may be preferably be employed.

The coloring reaction may be conducted employing chlorophenolred-β-D-galactopyranoside derivatives (CPRG) of the following formula as the substrate of β-D-galactosidase, and the change of the absorbance in the range of 574 nm about 30 nm is preferably measured.

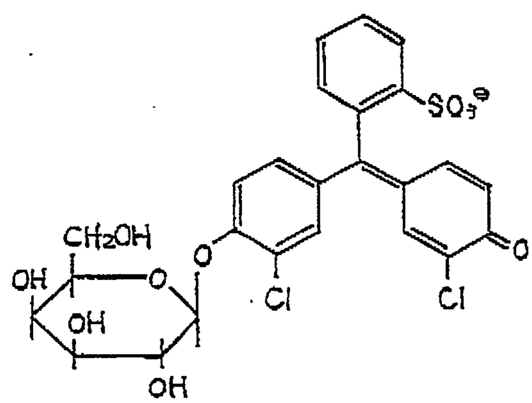

Further, the change of the signal originated from β-D-galactosidase may be detected utilizing the coloring reaction employing indolyl-β-D-galactoside derivatives. The indolyl-β-D-galactoside derivatives are known and are described in, for example, J. P. Horwitz et al., Journal of Medicinal Chemistry, vol. 7, pp. 574-575 (1964) and in Z. Lojda et al., Histochemie, vol. 34, pp. 361 - 369 (1973). Specific examples of the indolyl-β-D-galactopyranoside includes 3-indolyl-β-D-galactopyranoside, 5-bromo-3-indolyl-β-D-galactopyranoside and 5-bromo-4-chloro-3-indolyl-β-D-galactopyranoside, and the like.

To sensitively detect the signal originated from β-D-galactosidase, it is preferred to employ tetrazolium compound as a coloring agent. The tetrazolium compounds which may be employed in the present invention are described in, for example, M. M. Nachlas et al., Journal of Biological Chemistry, vol. 235, p.2739 (1960); A. G. E. Pearse et al., "Experientia" vol. 17, p. 136 (1961), Takuzo Oda et al, Okayama Medical Magazine, vol. 70, p. 128 (1958), M. M. Nachlas et al., Journal of American Chemical Society, vol. 78, p.6139 (1956); A. W. Sedar et al., Journal of Histochemistry and Cytochemistry, vol. 10, p. 506 (1962); and R. Gossrau, "Histochemistry", vol. 58, p. 203 (1978). Preferred specific examples of the tetrazolium compounds include 3-(p-iodophenyl)-2-(p-nitrophenyl)-5-phenyl-2-H tetrazolium chloride, 3-(4,5-dimethyl-2-thiazolyl)-2,5-diphenyl-2-H tetrazolium bromide, 3,3′-(4,4′-biphenylene)-bis(2,5-diphenyl-2-H tetrazolium chloride), 3,3′-(3,3′-dimethoxy-4,4′-biphenylylene)-bis[2-(p-nitrophenyl)-5-phenyl-2-H tetrazolium chloride, 3,3′-(3,3′-dimethoxy-4,4′-biphenylylene)-bis(2,5-diphenyl-2-H tetrazolium chloride) and 3,3′-(3,3′-dimethoxy-4,4′-biphenylylene)-bis[2,5-bis(p-nitrophenyl)-2-H tetrazolium chloride.

Further, as a catalyst of the coloring reaction, the substances described in, for example, S. Nakamura et al., "Clinica Chimica Acta, vol. 101, p.321 (1980); and in J. F. Burd etal., Clinica Chimica Acta" vol. 46, p.223 (1973) may be employed. Specific examples of the catalyst include 5-methylphenazinium methyl sulfate, (1-methoxy)-5-methylphenazinium methyl sulfate, 9-dimethylaminobenzo-α-phenazoxonium chloride and dihydrolipoamide reductase ($NAD^+$).

To aid the comrehension of the present invention, preferred embodiments will now be described with reference to the drawings.

Fig. 1 is a schematic view of the simplest mode of the assay element of the present invention. In this embodiment, the assay element comprises only the porous reaction layer 0, and the above-mentioned two kinds of carriers exist in the porous reaction layer.

Fig. 2 is a schematic view of the element shown in Fig. 1. In Fig. 2, the biological active substance, or biological substance-binding substance is fixed to the particles A, and the signal-changing substance is fixed to particles B. The particles C added for adjustment are also incorporated in the porous reaction layer. Fig. 2 shows that the particles A - C are point-adhered to form the porous reaction layer.

The mechanism of the method of the present invention wil now be described referring to Fig. 3. In Fig. 3, reference numeral 1 denotes the target substances, reference numeral 2 denotes β-D-galactosidasee, reference numeral 3 denotes a conjugate of the target substance and β-D-galactosidase (i.e., the labelled substance), reference numeral 4 denotes the substance which specifically binds to the target substance, reference numeral 5 denotes the biological active substance (or biological substance-binding substance), reference numeral 6 denotes the conjugate of the target substance which specifically binds to the target substance and the biological active substance, reference numeral 7 denotes the biological substance-binding substance (or biological active substance), reference numeral 8 denotes the signal-changing substance, reference numeral 9 denotes the reaction product (conjugate of the target substance 1 and the conjugate 6, reference numeral 10 denotes the reaction product (conjugate) of the labelled substance 3 and the conjugate 6, A denotes the carrier to which the biological substance-binding substance (or biological active substance) 7 is fixed, and B denotes the carrrier to which the signal-changing substance is fixed.

In operation, a certain amount of the fluid sample containing the target substance, a solution containing the labelled substance 3 and a solution containing the conjugate 6 is dropped on the porous reaction layer after mixing or in succession.

Since the target substance 1, labelled substance 3 and the conjugate 6 exist in the liquid phase, the binding reactions (1 and 6 or 3 and 6) are liquid phase reactions. Therefore, these reactions occur sufficiently more quickly than the binding reaction between the biological substance-binding substance (or biological active substance) 7 fixed to the carrier A, and the binding reaction between the labelled substance 3 and the signal-changing substance 8 fixed to the carrier B. Thus, after the reactions in the liquid phase are sufficiently conducted, the solid-liquid reactions occur. As a result of the liquid phase reactions, the non-reacted labelled substance 3 binds to the fixed signal-changing substance 8 on B (the binding site is 2 - 8), and the conjugate 9 binds to the substance 7 fixed on the carrier A (the binding site is 5 - 7). Although the binding reaction product 10 may bind to the fixed substance 7 or to the fixed signal-changing substance 8, by choosing the binding affinity between 5 and 7 is greater than the binding affinity between 2 and 8, it is possible to make the conjugate 10 preferentially binds to the fixed substance 7.

Thus, the reacted labelled substance 3 is bound to the carrier A and the non-reacted labelled substance 3 is bound to the carrier B, so that the separation of the reacted and non-reacted labelled substance (B/F separation) can be conducted in the same porous reaction layer. Since the target substance 1 and the labelled substance 3 competitively react with the conjugate 6, the amount of the non-reacted labelled substance 3 varies depending on the amount of the target substance 1 in the fluid sample. Since the signal originated from the β-D-galactosidase 2 is changed by the signal-changing substance 8, the intensity of the signal and the concentration of the target substance 1 has a functional relationship. Therefore, by preparing a calibration curve using several standard samples containing known concentration of the target substance 1, the concentration of the target substance 1 in an un-known sample can be determined using the calibration curve.

The mechanism in the so called sandwich method in which the a labelled substance which is a conjugate of the target-binding substance and β-D-galactosidase can be similarly explained.

In cases where at least one of the biological active substance and the biological substance-binding substance, which are fixed to the carrier is biotin or a derivative thereof, the biotin or the derivative thereof may be employed as the marker and in the measurement operation, avidin or a derivative thereof may be applied to the element together with the sample fluid or the labelled substance. This mode will now be

described referring to Fig. 3′.

In Fig. 3′, reference numeral 21 denotes the target substance, reference numeral 22 denotes the labelled substance, reference numeral 23 denotes the conjugate of the target substance and the marker (labelled substance), reference numeral 24 denotes the target-binding substance, reference numeral 25 denotes biotin or a derivative thereof, reference numeral 26 denotes a conjugate of the target-binding substance and biotin or a derivative thereof, reference numeral 27 denotes biotin or a derivative thereof, reference numeral 28 denotes the signal-changing substance, reference numeral 29 denotes the binding reaction product of the target substance and the conjugate 26, reference numeral 30 denotes the binding reaction product of the conjugate 23 and the conjugate 26, reference character A denotes a carrier to which the biotin 27 is fixed, reference character B denotes a carrier to which the signal-changing substance 28 is fixed, and reference numeral 31 denotes avidin or a derivative thereof.

In operation, a certain amount of the sample fluid, a solution containing the labelled substance 23 and a solution containing the conjugate 26 and avidin or a derivative thereof are dropped on the element after mixing or in succession.

Since the target substance 21, the labelled substance 23, the conjugate 26 and the avidin 31 exist in the liquid phase, the binding reaction of these reactants (the binding site is 21 - 26, 23 - 26, and 26 - 31) is a liquid phase reaction, so that these reaction occur suffiently quicker than the reaction between the immobilized biotin 27 and the avidin 31, or the reaction between the immobilized signal-changing substance 28 and the labelled substance 23. Thus, before the solid phase reactions occur, the liquid phase reactions are sufficiently conducted. As a result of the liquid phase reactions, the non-reacted labelled substance 23 in the liquid phase reacts with the signal-changing substance 28 fixed on the carrier B (the binding site is 2 - 8), the reaction product 29 reacts with the biotin 27 fixed on the carrier B through the avidin 31 (the binding site is 5 - 11 - 7). Although the binding reaction product 30 may bind to the biotin 27 or signal-changing substance 28, by choosing the binding affinity of 25 - 27 - 11 is greater than the binding affinity between 22 - 28, it is possible to preferentially make the labelled substance 23 or the reaction product 30 react with the biotin 27.

Thus, the reaction products 29 and 30 are bound to the carrier A through avidin 31, and the non-reacted labelled substance 23 is bound to the carrier B. Thus, the B/F separation can be attained in the reaction layer. Since the signal originated from the marker 22 is changed by the signal-changing substance 28, the concentration of the target substance in the sample fluid and the amount of the signal has a functional relationship. Therefore, by preliminarily preparing a calibration curve using standard samples containing known concentration of the target substance, the concentration of the target substance in an un-known sample may be determined.

Fig. 4 shows a schematic view of still another preferred embodiment of the assay element of the present invention. In this embodiment, the porous reaction layer is made of two sublayers and the biological active substance or the biological substance-binding substance and the signal-changing substance are fixed to the separate sublayers. These sublayers may be formed in the same manner as forming the porous reaction layer described above.

In addition to the indispensable porous reaction layer, to further promote the effect of the present invention, various supplemental layers may be employed. The embodiments employing these supplementa layers are schematically shown in Figs. 5 - 12.

The assay element 5 has a light-transmitting support 11 on which the porous reaction layer 0 is formed. Preferred examples of the material for constituting the support film include polymers such as cellulose acetate, polyethylene terephthalate, polycarbonate and polyvinyl compounds such as polystyrene, as well as inorganic materials such as glass. The reaction layer may directly be applied and/or formed into a film on the support. Alternatively, the support layer and the porous reaction layer are separately prepared and thereafter they may be laminated. In the embodiment shown in Fig. 5, the fluid sample is required to be dropped from the side of the reaction layer, but the measurement of the signal may be conducted from the either side of the element.

In the embodiment shown in Fig. 6, the porous reaction layer 0 is formed on a light-reflecting support 12. In using the element of this embodiment, the dropping of the fluid sample and the measurement of the signal are conducted from the side of the reaction layer, and the support 12 aids the measurement of the density of the reflection light. The support 12 may be made of a paper to which a water-proof treatment is performed, or may be made of the materials for forming the support 11 in Fig. 5. If necessary, to these materials, white pigment such as $TiO_2$, $BaSO_4$ and mica may be incorporated or applied.

The embodiment shown in Fig. 7 also aims the similar purpose and the porous reaction layer 0 and a light-reflecting layer 13 are laminated on a light-transmitting support 11. In using the element of this embodiment, the fluid sample is dropped from the side of the light-reflecting layer 13 and the measurement

of the signal is conducted from the side of the light-transmitting support 11. The light-reflecting layers which are employed in the conventional elements may be employed. Preferably, the particles and/or fibers for forming the porous reaction layer, in which the above-mentioned white pigments are incorporated are applied, formed into a film, or laminated. More preferably, to the particles containing the white pigments, the biological active substance or biological substance-binding substance, and the signal-changing substance are fixed, so that the light-reflecting layer has the similar function to the porous reaction layer below. By employing such a light-reflecting layer, it can be prevented that a large amount of the non-reacted labelled substance remain in the light-reflecting layer.

In the embodiment shown in Fig. 12, on the light-transmitting support 11, a coloring layer 15 is formed. The coloring layer 15 is made of at least one layer of hydrophilic colloid containing the coloring agent and the substrate which are required for the measurement of the enzyme activity. The coloring agent and the substrate may be soluted or dispersed in a binder made of hydrophilic colloid to prepare a coating solution for forming the coloring layer 15. Especially, for dispersing hydrophobic compounds, the conventional techniques widely used in the field of photography, such as the oil-protecting dispersion technique, or direct dispersion technique may be employed. As the hydrophilic colloid for forming the coloring layer 15, gelatin or derivatives thereof such as phthalic gelatin,; synthetic polymers such as polyvinyl alcohol, polyvinylpyrrolidone, polyvinyl imidazole, polyacrylamide and sodium polyacrylate; and cellulose derivatives such as hydroxyethyl cellulose and sodium carboxymethyl cellulose may be employed. Among these, the preferred are gelatin and phthalic gelatin.

In order to improve the physical properties of the coloring layer such as degree of swelling and fusibility by heat, part of the macromolecular substance may be replaced with water-dispersible polymer such as macromolecular latex. Preferred examples of the macromolecular latex include those described in Japanese Patent Disclosure (Kokai) Nos. 116258/82 and 99752/83. Although 70% by weight of the hydrophilic colloid binder may be replaced with the macromolecular latex, it is preferred that the percentage of the hydrophilic colloid replaced with the macromolecular latex be not more than about 50% by weight.

The coloring layer, as well as other layers of the element may optionally contain other additives such as a buffering agent, preservative, surface active agent and mordant. The buffering agent which may be contained in the coloring layer or other layers serves to make and keep the pH in the layer in the range preferred for the coloring reaction or enzyme reaction. The buffering agents which may be employed are described in "Manual of Chemistry, Basic Edition" , 1966, pp.1312-1320, edited by Japan Chemistry Association, published by Maruzen Co., Ltd., Tokyo; N. E. Good et al., Biochemistry, vol. 5, p.467 (1966); Imamura and Saito, "Domain of Chemistry", vol 30 (2), p. 79 (1976); W. J. Ferguson et al., Anal. Biochem., vol. 104, p.300 (1980). Specific examples of the buffering agents which may be employed may include salts of citric acid, boric acid, phosphoric acid, tris(hydroxymethyl)aminomethane, barbital, glycine and Good's buffering agent. The content of the buffering agent may be appropriately selected. These buffering agent may also be contained in the layers other than the coloring layer. As the preservative and the hardening agent which may be incorporated in the coloring layer, the substances described in "Biochemistry Experiment 1, Chemistry of Proteins I", 1976, edited by Japan Biochemistry Association and published by Tokyo Kagaku Dojin), pp.66-67, "Experiment and Application, Affinity Chromatography" published by Kodansha Scientific, 1976 pp. 16 - 104, and Japanese Patent Disclosure (Kokai) No. 149927/85 may be employed. Specific examples of these substances may include gelatin, decomposition products of gelatin, albumin, cyclodextrin, non-reduced saccharides (such as sucrose, trehalose), polyethyleneglycol, amino acids such as glycine, glycylglycine, various ions such as ammonium sulfate and sodium azide. The content of these substances in the coloring layer may be appropriately selected.

The coloring layer made of a hydrophilic colloid such as gelatin and gelatin derivative may also contain a hardening agent. The hardening agent widely used in the field of photograph may be employed. The hardening agents which may be employed are described in T. H. James ed., "The Theory of the Photographic Prorcess", 4th ed., pp. 77-87. That is, aldehydes, active olefins and active esters may be employed as the hardening agent. The content of the hardening agent in the coloring layer is usually 10 $mg/m^2$ to 300 $mg/m^2$.

Preferred examples of the surface active agent which may be incorporated in the coloring layer or in any other layers of the element include non-ionic and ionic surface active agents such as octylphenoxypolyethoxyethanol (commercially available from Rohm and Haas under the trade name of Triton X-100), nonylphenoxypolyglycidol (commercially available from Oline Co., Ltd. under the tradename of Surfactant 10G). The mordant which may be incorporated in the coloring layer serves to intensively collect the detection product required for the measurement of the enzyme activity and, in the case where the detection product is a dye to promote the absorbance or to shift the wavelength. Preferred mordant may include polymers such as described in Japanese Patent Disclosure (Kokai) No. 87461/83 and latex of these

polymers.

In the above-mentioned embodiments of the assay element of the present invention, the conjugate of the target-binding substance and the biological active substance of the biological substance-binding substance (hereinafter referred to as "the conjugate" for short) and the labelled substance are not contained in the element. These modes in which the conjugate and the labelled substance are not contained in the element have great freedom free from the restraint by the conjugate and the labelled substance. These modes have a surprising advantage as follows:

That is, all of the conventional immunoassay elements are for assaying a certain substance. That is, the immunoassay element prepared for the measurement of a target substance $M_1$ cannot be used for assaying a target substance $M_2$. Therefore, to assay a plurality of target substances in a single sample, the same number of the elements as the target substances must be prepared and an element for a certain target substance must be selected in operation. In general, to regularly keep and administer many assay elements which have term for validity is troublesome and not economical. Further, selecting an element in each assay reduces the efficiency of the operation and also jeopardize the correctness of the measurement due to the mis-selection. Further, in automating the operation, since the same number of cartridges as the number of the elements must be provided and a mechanism for switching the cartridges must be provided, so that the apparatus is inevitably made large and expensive. These drawbacks in the conventional elements reduces the simplicity of the operation in dry chemistry methods. On the other hand, in using the above-described embodiments of the assay element of the present invention, which do not contain the conjugate and the labelled substance, by selecting the conjugate and the labelled substance, and by dropping these with the fluid sample, an optional target substance $M_i$ can be assayed. Thus, these modes of elements may be used for any target substance. Further, by adjusting the amount of the conjugate and the labelled substance, the sensitivity of the element and the measurement range can be changed.

It should be noted, however, the element of the present invention is not restricted to such a multipurpose type. To simplify the assaying operation, the conjugate and the labelled substance may be contained in the assay element. By using this mode of the element in which the conjugate and the labelled substance are incorporated (this type of element will be hereinafter referred to as "incorporation type" for short), although the target substance to be measured by the element is restricted, the operation to drop the conjugate and the labelled substance may be omitted. Thus, depending on the desire of the user, both of the multipurpose type and incorporation type may be selected, which is an important feature of the present invention..

The embodiment of the incorporation type will now be described referring to the accompanying drawings.

In the embodiment shown in Fig. 8, a coloring layer 15 is formed on the light-transmitting support, and a conjugate-containing layer 14 is formed thereon. In the conjugate-containing layer, the conjugate is incorporated in a porous medium such that the area concentration of the conjugate is uniform. Therefore, when a certain amount of the fluid sample and a solution containing the labelled substance are dropped, a certain amount of the conjugate is eluted and diffused to the porous reaction layer together with the fluid sample. As the material constituting the conjugate-containing layer, the above-mentioned materials for constituting the porous reaction layer may be employed, and the materials may be applied, formed into a film or laminated. Alternatively, pulp, cellulose powder and natural, synthetic and semisynthetic fibers of various plants, animals, minerals and artificial products such as cotton, hemp, silk, wool, chitin, chitosan, cellulose ester, viscose rayon, copper-ammonia rayon, polyamides (e.g., 6-Nylon, 6,6- Nylon and the like), polyesters (e.g., polyethylene terephthalate and the like), polyolefins (polypropyrene, vinylon and the like), glass and asbestos may be employed. These materials may be employed individually or in combination. Further, water-absorptive European paper, Japanese paper, filter paper and brush polymer, as well as the woven fabrics, non-woven fabrics and synthetic paper made from one or more of the above-described fibers may also be employed.

The position of the conjugate-containing layer is not restricted to the uppermost position. In cases where the conjugate-containing layer is not in the uppermost position, the materials constituting the conjugate-containing layer may be, in addition to those described above, hydrophilic macromolecules such as gelatin, gelatin derivatives, polysaccharides (e.g., agarose), carboxymethyl cellulose and hydroxyethyl cellulose, as well as homopolymers or copolymers of vinyl pyrrolidone derivatives, methacryl acid derivatives, vinyl alcohol and sulfonyl styrene. These may be used as a uniform binder and may be applied.

In the embodiment shown in Fig. 9, the labelled substance is also contained in the element. Although the layered structure is the same as the embodiment shown in Fig. 8, the labelled substance is incorporated in the porous reaction layer. In the porous reaction layer, the signal-changing substance which specifically binds to β-D-galactosidase in the labelled substance is fixed. To prevent the binding reaction between the

labelled substance and the signal-changing substance, a method described in Clinical Chemistry, vol. 27, p. 1499 (1981) may be employed.

In another mode of the element containing the conjugate and the labelled substance, the conjugate is incorporated in the porous reaction layer and the labelled substance is incorporated in the conjugate-containing layer in Fig. 9. In this case, the labelled substance-containing layer may be formed as in forming the conjugate-containing layer, and the conjugate may be incorporated in the porous reaction layer in the same manner as in incorporating the labelled substance therein.

The conjugate and the labelled substance may also be incorporated in the porous reaction layer.

Fig. 10 and 11 are a cross-sectional view and a perspective view, respectively, of a preferred embodiment of the assay element of the present invention. In this embodiment, for the purpose of making it easy to handle the element, the entire element is placed in a plastic mount 17, and a fluid sample injection hole and a signal measuring hole are formed in the upper and lower portion of the mount 17, respectively.

The element of the present invention may further comprise a spreading layer which aids the spreading of the sample fluid when it is dropped on the element. Further, the conventional supplemental layers such as blood-separating layer which is necessary when the fluid sample is blood (whole blood), adhesive layer, protection layer and timing layer may be formed.

These supplemental layers and the above-described coloring layer, conjugate- or labelled substance-containing layer, and the timing layer may be formed individually or a layer having two or more functions in combination may be formed. The position of these layers may easily be determined by those skilled in the art depending on the function thereof.

In another aspect of the present invention, a method of assaying a target substance comprising the steps of reacting a) the target substance in the fluid sample, b) a labelled substance which is the target substance or an analogue thereof labelled with β-D-galactosidase, or a labelled substance which is a substance which specifically binds to the target substance, labelled with -D-galactosidase, c) the substance which specifically binds to the target substance, which is fixed to a carrier contained in a porous reaction layer of an immunoassay element, and d) a substance which specifically binds to β-D-galactosidase and which changes a signal originated from β-D-galactosidase, which is fixed to said carrier or another carrier which exists in a porous reaction layer of an assay element; and measuring the change of the signal from β-D-galactosidase, thereby detecting the existence of the target substance or determining the amount of the target substance in the fluid sample, is employed. In this method, the target substance and the labelled substance competitively reacts with the target-binding substance b) fixed to a carrier contained in a porous reaction layer of an immunoassay element. Therefore, the greater the amount of the target substance contained in the fluid sample, the more the non-reacted labelled substance. Therefore, as in the above-described method of the present invention, the change of the signal from β-D-galactosidase is dependent on the target substance in the fluid sample, so that by measuring the change of the signal, the amount of the target substance in the fluid sample can be determined. As to other respects of this method, the discussion about the above-described method of the present invention can be applied.

As mentioned above, one of the preferred signal-changing substance is aryl mercury derivatives. However, if aryl mercury derivatives are reacted under the presence of a buffer widely used in this field such as phosphate buffer, tris(hydroxymethyl)aminomethane, glycine and citric acid, the enzyme inhibitory activity of the aryl mercury derivatives is sharply decreased, so that the reliability of the assay is reduced. The present inventors have found that the aryl mercury derivatives are used in the presence of the compound of the formula [I] or [II] or a derivative thereof.

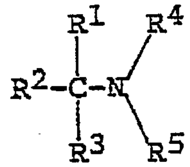

[I]

(wherein $R^1$, $R^2$, $R^3$, $R^4$ and $R^5$, the same or different, represent $C_1$ - $C_5$ alkyl group which has a hydroxide group or a sulfonyl group as a substituent on the main chain thereof).

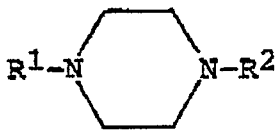

[II]

(wherein $R^1$ and $R^2$, the same or different, represent the same meaning as in formula [I]).

Specific examples of the compounds represented by the above formula [I] include the following:

$(HOCH_2)_3CN(CH_2CH_2OH)_2$

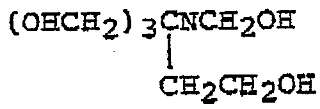

$(OHCH_2)_3CN(CH_2CH_2SO_3H)_2$

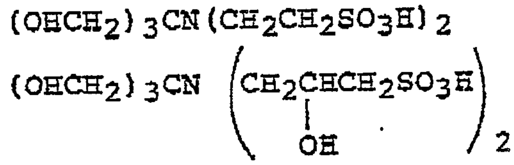

$(HOCH_2CH_2)_3CN(CH_2OH)_2$

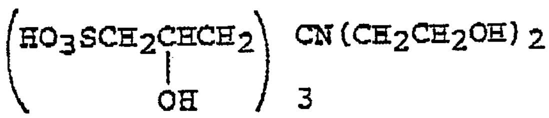

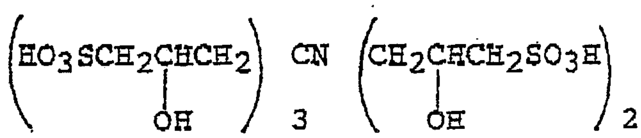

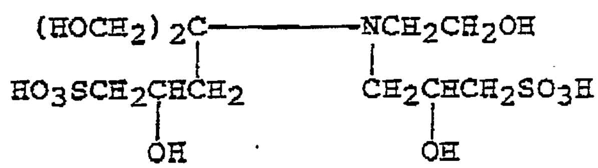

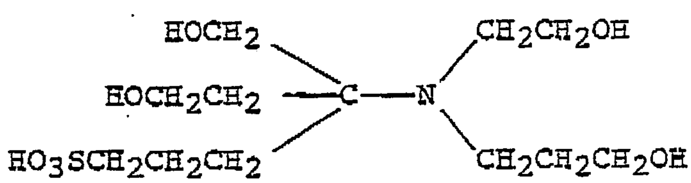

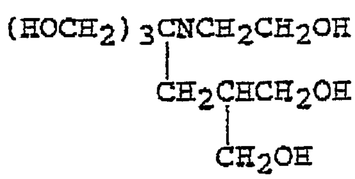

Among the compounds represented by the formula [I], bis(2-hydroxyethyl)iminotris(hydroxymethyl)-methane is especially preferred.

These compounds may easily be prepared by the method described in Archives of Biochemistry and Biophysics, vol. 96, p653 (1962); or Analytical Biochemistry, vol. 104 p. 300 (1980).

The compounds of the formula [I] wherein $R^1$, $R^2$ and $R^3$ are $-CH_2OH$ are easy to manufacture using tris(hydroxymethyl)aminomethane which is widely used in this field as a starting material.

Specific examples of the compounds represented by the formula [II] may include the following:

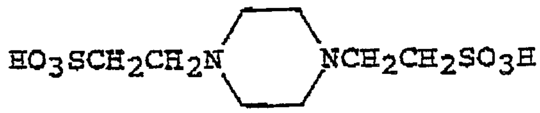

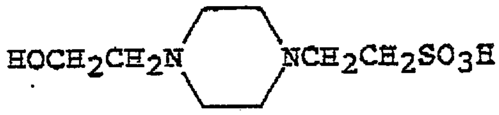

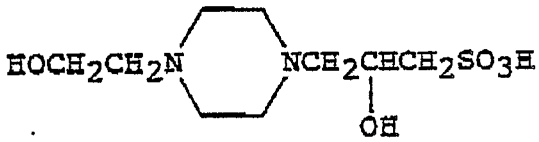

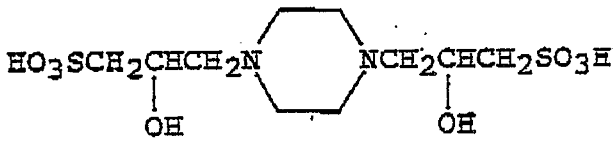

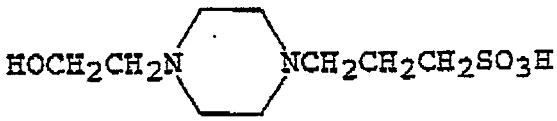

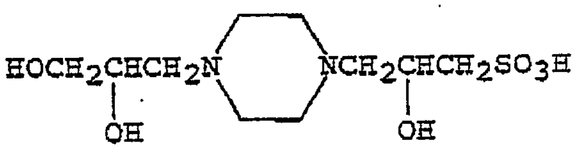

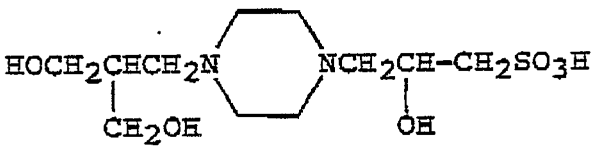

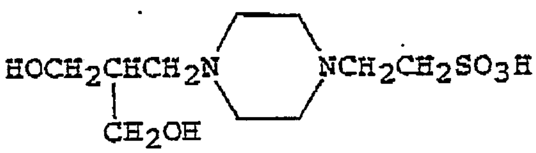

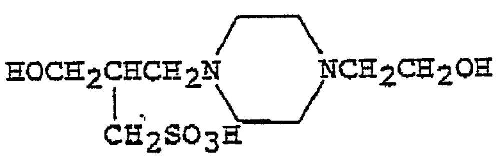

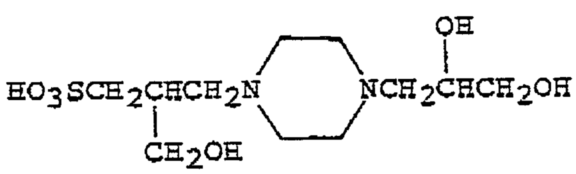

Among these, piperazine-N,N′-bis(2-ethanesulfonic acid) and N-2-hydroxyethylpiperazine-N′-2-ethane sulfonic acid are especially preferred.

These compounds may easily be prepared by the method described in Archives of Biochemistry and Biophysics, vol. 96, p653 (1962); or Analytical Biochemistry, vol. 104 p. 300 (1980).

The buffer containing the compound of the formula [I] or II] preferably has a pH of 6.5 - 8.5, more preferably 7.0 - 7.5. The pH may be adjusted by using, for example, a strong base such as sodium hydroxide and potassium hydroxide, or a strong acid such as hydrochloric acid to partly neutralize the compound.

Although the concentration of the compound of the formula [I] or [II] in the buffer at the time of measurement varies depending on the sample and the assay system, usually, the concentration is preferably 0.001 -10 M, more preferably 0.01 - 1 M.

Further, the buffer containing the compound of the formula [I] or [II] may preferably contain $Mg^{2+}$ ion in the amount of 0.1 - 100 mM at the time of measurement.

The compound of the formula [I] or [II] may be admixed with the fluid sample or may be incorporated in the assay element. In cases where the compoud of the formula [I] or [II] is incorporated in the element, the above-described method of incorporating the buffer in the coloring layer or in other layers may be employed.

The preferred thickness of each layer constituting the assay element is determined not only by the function of the layer, but also the material for constituting the layer and also the existence of the other layers.

In cases where the porous reaction layer which is indispensable to the assay element of the present invention is mainly composed of particles, the thickness of the porous reaction layer may preferably be 3 - 700 µm, more preferably be 10 -300 µm. Further, in cases where the porous reaction layer has a plurality of sublayers mainly composed of particles, or in cases where the element comprises other functional layers such as spreading layer, which are mainly composed of particles, the total of the thickness of the layers mainly composed of particles may preferably be 10 - 700 µm, more preferably 20 - 300 µm.

In cases where the porous reaction layer is mainly composed of fibers, the thickness of the porous reaction layer may preferably be 10 - 700 µm, more preferably 20 -300 µm. Further, in cases where the element comprises other functional layers such as spreading layer, which are mainly composed of fibers, the total thickness of the layers mainly composed of the fibers may preferaby 40 -1200 µm, more preferably 100 - 400 µm.

In cases where the element has both the porous layer mainly composed of particles and the porous layer mainly composed of fibers, the total thickness of the porous layers may preferably be 60 - 1500 µm, more preferably 100 - 900 µm.

In cases where the element has a layer such as coloring layer, conjugate-containing layer or labelled substance-containing layer, which is composed of a continuous binder, the thickness of such a layer may preferably be 5 - 200 µm, more preferably 10 - 100 µm. Further, the total thickness of these layers may also preferably be 5 - 200 µm, more preferably 10 - 100 µm.

As to the adhering layer, protection layer and the timing layer, the thickness thereof is preferably as small as possible, as long as the layer functions, and there is no lower limit of the layer.

The invention will now be described referring to the examples thereof. The examples are presented for the illustration purpose only and should not be interpreted any restrictive way.

Example 1

1 - (1) Preparation of p-aminophenyl Mercuric Acetate (Enzyme Inhibitor) Fixed to Microcrystalline Cellulose

Eighty grams of microcrystalline cellulose (commercially available from Asahi Chemicals Inc., Osaka, Japan under the tradename of Avicel) was suspended in 800 ml of 2.5 M phosphate buffer (pH 12.0). To this suspension, 80.0 g of cyanogen bromide cyanide soluted in 800 ml of distilled water was added and the resulting mixture was allowed to react for 20 minutes while cooling in iced water. The thus treated Avicel was recovered by filtration and was sufficiently washed with water. The recovered 80 g of Avicel was suspended in 950 ml of 25% aqueous dimethyl sulfoxide solution containing 4.8 g of p-aminophenyl mercuric acetate and the resulting mixture was stirred at room temperature for 20 hours. The thus treated Avicel was removed by filtration and was washed with dimethyl sulfoxide and distilled water. The Avicel was then suspended in 1000 ml of 1 M tris-HCl buffer (pH 8.5) and the suspension was stirred at room temperature for 20 hours to block the non-reacted groups. The Avicel was removed by filtration and was sufficiently washed with water. It was then washed with acetone and was dried to obtain Avicel to which p-aminophenyl mercuric acetate was fixed.

1-(2) Preparation of Avidin-Fixed Avicel

In 330 ml of 0.01 M phosphate buffer (pH 7.4) containing 0.15 M sodium chloride, 1.0 g of bovine serum albumin (fraction V, manufactured by Arma Co., Ltd., U.S.) was dissolved. To this solution, 10.4 mg of biotin-N-hydroxy succinimide ester (manufactured by Boehringer) in 3.0 ml of dimethylformamide was added and the resulting mixture was allowed to react at room temperature for 2.5 hours. The resulting reaction mixture was sufficiently dialyzed against the same buffer and was lyophilized to obtain bovine serum albumin to which biotin was bound. In 1800 ml of 2.5 M phosphate buffer (pH 12.0), 90 g of Avicel was suspended, and 45.0 g of cyanogen bromide in 550 ml of distilled water was added thereto while cooling the suspension in iced water. After allowing to react for 20 minutes, the Avicel was removed by filtration and was sufficiently washed with water. Ninety grams of this Avicel was suspended in 900 ml of 0.1 M sodium hydrogen carbonate solution containing 500 mg of the bovine serum albumin to which biotin was bound and containing 0.15 M sodium chloride, and the suspension was stirred at 4°C for 20 hours. The thus treated Avicel was removed by filtration and was alternately washed with distilled water, 0.1 M aqueous sodium hydrogen carbonate solution containing 0.15 M sodium chloride, and 0.1 M sodium acetate buffer (pH 4.1) containing 0.15 M sodium chloride, and was suspended in 1200 ml of 1 M Tris-HCl buffer (pH 8.5). The resulting suspension was stirred at room temperature for 20 hours to block the non-reacted reactive groups. The thus treated Avicel was removed by filtration and was sufficiently washed with water. The Avicel was then suspended in 450 ml of 0.05 M phosphate buffer (pH 7.4) containing 270 mg of avidin (manufactured by Oriental Yeast Co., Ltd.) and 0.15 M sodium chloride and the suspension was allowed to react at 4°C for 20 hours. The thus treated vicel was removed by filtration and was washed with water to obtain Avicel to which avidin was bound. Eighty grams of this avidin-bound Avicel was suspended in 240 ml of distilled water containing 15.4 g of bovine serum albumin and 9.91 g of sucrose. The suspension was freeze-dried to obtain avidin-bound Avicel containing bovine serum albumin and sucrose.

1-(3) Preparation of Human IgG Labelled with β-D-galactosidase

Twenty milligrams of human IgG (manufactured by Cappel Co., Ltd., U.S.) was dissolved in 2.0 ml of 0.1 M phosphate buffer (pH 6.5), and 77 µl of 2.5 mg/ml solution of N-(ε-maleimidocaproyloxy)succinimide (manufactured by Dojindo Laboratories) in dimethylformamide was added thereto. The resulting mixture was allowed to react at 30°C for 20 minutes and the resultant was purified with Sephadex G-25 column (commercially available from Pharmacia) equilibrated with 0.1 M phosphate buffer (pH 6.0) containing 5 mM EDTA to obtain human IgG modified with maleimide. To 1.8 ml of 10.5 mg/ml solution of β-D-galactosidase (manufactured by Toyobo Co., Ltd.) in 0.1 M phosphate buffer, 3.2 ml of a solution containing 13.6 mg of the human IgG modified with maleimide obtained above was added, and the resulting mixture was allowed to react at 4°C for 45 hours. Thereafter, 175 µl of 0.1 M 2-mercaptoethylamine was added to the mixture and was allowed to react at 30°C for 20 minutes. The resultant was purified with Superose 6 Prep grade column (manufactured by Pharmacia) equilibrated with 0.15 M sodium chloride-containing 0.1 M phosphate buffer (pH 7.4) to obtain human IgG labelled with β-D-galactosidase.

1-(4) Preparation of Biotin-bound Goat Anti-human IgG Antibody

In 2.0 ml of 0.1 M phosphate buffer (pH 7.4) containing 0.15 M sodium chloride, 5.8 mg of goat anti-human IgG antibody (manufactured by Cappel Co., Ltd., U.S.) was dissolved. To this solution, 500 µl of a solution in dimethylformamide containing 0.32 mg of biotin-N-hydroxy-succinimidester (manufactured by Boehringer) was added, and the resulting mixture was allowed to react at room temperature for 3 hours. The resultant was sufficiently dialyzed against 0.01 M phosphate buffer containing 0.15 M sodium chloride to obtain goat anti-human IgG antibody to which biotin was attached.

1-(5) Preparation of Immunoassay Element

On a transparent polyethylene terephthalae film with a thickness of 180 µm, a coating solution (1) with the following composition was applied and dried to form a gelatin layer.

| Composition of Coating Solution (1) | |
|---|---|
| Deionized Gelatin | 6.0 g |
| Triton X-100 (manufactured by Room and Haas) | 0.15 g |
| 1,2-bis(vinylsulfonyl)ethane | 0.01 g |
| Distilled Water | 54.0 g |

Then a coating solution (2) containing a coloring agent and in which the p-aminophenylmercuric acetate-bound Avicel obtained in 1-(1) was dispersed was applied on the gelatin layer and dried.

| Composition of Coating Solution (2) | |
|---|---|
| p-aminophenylmercuric acetate-bound Avicel | 14.0 g |
| Triton X-100 | 1.4 g |
| Polyvinylpyrrolidone (Wako Pure Chemicals) | 1.2 g |
| 5-bromo-4-chloro-3-indolyl-β-D-galactopyranoside (Boehringer) | 0.90 g |
| NeoTB* (Dojindo Laboratories) | 0.35 g |
| n-butanol | 34.0 g |

*NeoTB; 3,3′-(4,4′-biphenylene)-bis(2,5-diphenyl-2H-tetrazolium chloride)

Then a coating solution (3) with the following composition in which the avidin-bound Avicel obtained in 1-(2) was dispersed was applied on the above-described layer containing p-aminophenylmercuric acetate-bound Avicel, and dried.

| Composition of Coating Solution (3) | |
|---|---|
| Avidin-bound Avicel containing bovine serum albumin and sucrose | 22.5 g |
| Triton X-100 | 2.50 g |
| Polyvinylpyrrolidone | 3.50 g |
| n-butanol | 52.0 g |

A coating solution (4) with the following composition was applied on the above-described avidin-bound Avicel layer and dried to form a spreading layer.

Composition of Coating Solution (4)

| | |
|---|---|
| Cellulose powder D (Toyo Filter Paper Co., Ltd.) | 30.0 g |
| Triton X-100 | 3.0 g |
| Polyvinylpyrrolidone | 1.4 g |
| n-butanol | 80.0 g |

The thus prepared sample was cut into pieces sizing 1.5 cm x 1.5 cm to obtain an immunoassay element 1 of the present invention.

1-(6) Measurement of Human IgG

Human IgG (0 - 640 μg/ml) dissolved in 0.3 M bis(2-hydroxyethyl)iminotris(hydroxymethyl)methane (hereinafter referred to as "Bistris" for short)-HCl buffer (pH 7.2) containing 1 mM of magnesium chloride

and 6 wt% of bovine serum albumin was mixed with the human IgG labelled with $\beta$-D-galactosidase (10 $\mu$g/ml solution in the same buffer) prepared in 1-(3) and biotin-bound anti-human IgG antibody (20 $\mu$g/ml solution in the same buffer). Ten microliters of this mixture was dropped on the immunoassay element 1 prepared in 1-(5) and the element was incubated in sealed condition at 37° C for 10 minutes. Thereafter, the reflection density at 546 nm was measured from the side of the support layer. The results are shown in Fig. 13.

## Example 2

### 2-(1) Preparation of Avidin-bound Cellulose Fibers

The same procedure as in 1-(2) was repeated except that Cellulose Powder D was used instead of Avicel of avidin-bound Avicel to prepare avidin-bound cellulose fibers containing bovine serum albumin and sucrose.

### 2-(2) Preparation of Immunoassay Element

On a transparent polyethyleneterephthalate film with a thickness of 180 $\mu$m, a coating solution (5) with the following composition was applied and dried to form a gelatin layer.

| Composition of Coating Solution (5) | |
|---|---|
| Deionized Gelatin | 4.5 g |
| Triton X-100 (manufactured by Room and Haas) | 0.10 g |
| 1,2-bis(vinylsulfonyl)ethane | 0.007 g |
| NeoTB | 0.23 g |
| Distilled Water | 54.0 g |

Then a dispersion with the same composition as the coating solution (2) described in 1-(5) except that the dispersion does not contain NeoTB was applied on the gelatin layer and dried to form p-aminophenylmercuric acetate-bound Avicel layer.

On this layer, the coating solution (6) with the following composition was applied and dried. The sample was then cut into pieces sizing 1.5 cm x 1.5 cm to obtain an immunoassay element 2 of the present invention.

| Coating Composition (6) | |
|---|---|
| Avidin-bound Cellulose Powder D containing bovine serum albumin and sucrose | 30.0 g |
| Triton X-100 | 3.0 g |
| Polyvinylpyrrolidone | 1.5 g |
| n-butanol | 55.0 g |

### 2-(3) Measurement of Human IgG

Human IgG was measured by the same manner as in 1-(6). The results are shown in Fig. 14.

## Example 3

### 3-(1) Preparation of Immunoassay Element

On a polyethyleneterephthalate film with a thickness of 180 µm, the coating solution (5) described in 2-(2) was applied and dried to form a gelatin layer. Then a dispersion with the same composition as the coating solution (2) described in 1-(5) except not containing NeoTB was applied and dried to form p-aminophenylmercuric acetate-bound Avicel layer.

On this layer, a coating solution (7) with the following composition containing the human IgG labelled with β-D-galactosidase prepared in 1-(3) and the biotin-bound goat anti-human IgG antibody prepared in 1-(4) was applied and dried. Further, on this layer, the coating solution (6) described in 2-(2) was applied and dried. The sample was then cut into pieces sizing 1.5 cm x 1.5 cm to obtain an immunoassay element 3 of the present invention.

| Composition of Coating Solution (7) | |
|---|---|
| Avidin-bound Avicel containing bovine serum albumin and sucrose | 22.5 g |
| Triton X-100 | 2.50 g |
| Polyvinylpyrrolidone | 3.30 g |
| Human IgG labelled with β-D-galactosidase | 2.0 mg |
| Biotin-bound goat anti-human IgG antibody | 2.0 mg |
| Bovine serum albumin | 38.0 mg |
| n-butanol | 55.0 mg |

3-(2) Measurement of Human IgG

Human IgG (0 - 640 µg/ml) dissolved in 0.3 M Bistris-HCl buffer (pH 7.2) containing 1 mM magnesium chloride and 6 wt% of bovine serum albumin was dropped on the immunoassay element 3 and the element was incubated in sealed condition at 37° C for 10 minutes. Reflection density was measured at 546 nm from the side of the support layer. The results are shown in Fig. 15.

## Example 4

4-(1) Preparation of Rabbit Anti-human IgG Antibody Labelled with β -D-galactosidase

In the same manner as in 1-(3) using 20 mg of rabbit anti-human IgG antibody (manufactured by Cappel), rabbit anti-human IgG antibody labelled with β -D-galactosidase was obtained.

4-(2) Measurement of Human IgG

Human IgG (0 - 640 µg/ml) dissolved in 0.3 M Bistris-HCl buffer (pH 7.2) containing 1 mM magnesium chloride and 6 wt% of bovine serum albumin, rabbit anti-human IgG antibody labelled with β -galactosidase (15 µg/ml solution in the same buffer), and biotin-bound goat anti-human IgG antibody (100 µg/ml solution in the same buffer) were mixed and 10 µl of the mixture was dropped on the element 1 prepared in 1-(5). The element was incubated in sealed condition at 37° C for 10 minutes and the reflection density was measured from the side of the support layer at 546 nm. The results are shown in Fig. 16.

As is apparent from Figs. 13 - 16, by using the element of the present invention, B/F separation can be performed in the element and human IgG can be measured with high sensitivity.

## Example 5

Human IgG (0 - 640 µg/ml) dissolved in various 0.3 M solutions (pH 7.2) summerized in Table 2 below containing 1 mM of magnesium chloride and 6 wt% of bovine serum albumin was mixed with the human IgG labelled with β-D-galactosidase (10 µg/ml solution in the same buffer) prepared in 1-(3) and biotin-bound anti-human IgG antibody prepared in 1-(4) (20 µg/ml solution in the same buffer). Ten microliters of this mixture was dropped on the immunoassay element 1 prepared in 1-(5) and the element was incubated

in sealed condition at 37°C for 10 minutes. Thereafter, the reflection density at 546 nm was measured from the side of the support layer. The results are shown in Figs. 17 and 18.

Example 6

The same procedure as in Example 5 was repeated except that the tested element was element 2 obtained in Example 2. The results are shown in Figs. 19 and 20.

Example 7

7-(1) Preparation of Immunoassay Element

The same procedure as in 3-(1) was repeated except that a coating solution (8) with the following composition was used instead of the coating solution (7) to form an immunoassay element 5.

| Composition of Coating Solution (8) | |
|---|---|
| Avidin-bound Avicel containing bovine serum albumin and sucrose | 22.5 g |
| Triton X-100 | 2.50 g |
| Polyvinylpyrrolidone | 3.30 g |
| Human IgG labelled with β -D-galactosidase | 0.16 mg |
| Biotin-bound goat anti-human IgG antibody | 0.60 mg |
| Bovine serum albumin | 38.0 mg |
| n-butanol | 55.0 g |

7-(2) Measurement of Human IgG

Human IgG (0 - 640 μg/ml) dissolved in various 0.3 M solutions (pH 7.2) summerized in Table 2 below containing 1 mM of magnesium chloride and 6 wt% of bovine serum albumin was dropped on the thus prepared element 5. The element was then incubated in sealed condition at 37°C for 10 minutes, and then the reflection density was measured at 546 nm from the side of the support layer. The results are shown in Figs. 21 and 22.

Example 8

Human IgG (0 - 640 μg/ml) dissolved in various 0.3 M solutions (pH 7.2) summerized in Table 2 below containing 1 mM of magnesium chloride and 6 wt% of bovine serum albumin was mixed with the human IgG labelled with β-D-galactosidase (10 μg/ml solution in the same buffer) prepared in 1-(3) and biotin-bound anti-human IgG antibody prepared in 1-(4) (20 μg/ml solution in the same buffer). Ten microliters of this mixture was dropped on the immunoassay element 1 prepared in 1-(5) and the element was incubated in sealed condition at 37°C for 10 minutes. Thereafter, the reflection density at 546 nm was measured from the side of the support layer. The results are shown in Figs. 23 and 24.

Example 9

9-(1) Preparation of Goat Anti-human IgG Antibody-bound Avicel

In 1800 ml of 2.5 M phosphate buffer (pH 12.0), 90 g of Avicel was suspended, and 45.0 g of cyanogen bromide dissolved in 550 ml of pure water was added thereto. After allowing to react for 20 minutes, Avicel was removed by filtration and was sufficiently washed with water. Ninety grams of this Avicel was

suspended in 900 ml of 0.1 M aqueous sodium hydrogen carbonate solution containing 600 mg of goat anti-human IgG antibody (Cappel) and containing 0.15 M sodium chloride. This suspension was stirred to react at 4° C for 20 hours. The thus treated Avicel was removed by filtration and was alternately washed with pure water, 0.1 M aqueous sodium hydrogen carbonate solution containing 0.15 M sodium chloride and 0.1 M sodium acetate buffer (pH 4.1) containing 0.15 M sodium chloride, and was suspended in 1200 ml of 1 M Tris-HCl buffer (pH 8.5). The suspension was stirred at room temperature for 20 hours to block the non-reacted reactive groups. After removing the thus treated Avicel by filtration and after sufficient washing with water, 80 g of the antibody-bound Avicel was suspended in 240 ml of pure water containing 15.4 g of bovine serum albumin and 9.91 g of sucrose. The resulting suspension was freeze-dried to obtain goat anti-human IgG antibody-bound Avicel containing bovine serum albumin and sucrose.

9-(2) Preparation of Immunoassay Element

The same procedure as in 1-(5) was repeated except that 22.5 g of the goat anti-human IgG antibody-bound Avicel containing bovine serum albumin and sucrose prepared in 9-(1) was used in place of the avidin-bound Avicel in the coating solution (3), to prepare an immunoassay element 6.

9-(3) Measurement of Human IgG

Human IgG solutions (0 - 640 μg/ml) in various 0.3 M buffers (pH 7.2) shown in Table 2 below containing 1 mM magnesium chloride and 6 wt% of bovine serum albumin were mixed with human IgG labelled with β-D-galactosidase (10 μg/ml solution in the same buffer) obtained in 1-(3). Ten microliters of this mixture was dropped on the immunoassay element 6 obtained in 9-(2), and the element was incubated in sealed condition at 37° C for 10 minutes. Thereafter, the reflection density was measured at 546 nm from the side of the support layer. The results are shown in Figs. 25 and 26.

Example 10

Measurement of Human IgG

Human IgG solutions (0 - 640 μg/ml) in various 0.3 M buffers (pH 7.2) shown in Table 2 below containing 1 mM magnesium chloride and 6 wt% of bovine serum albumin were mixed with rabbit anti-human IgG antibody obtained in 8-(1) (10 μg/ml solution in the same buffer). Ten microliters of this mixture was dropped on the immunoassay element 6 obtained in 9-(2), and the element was incubated in sealed condition at 37° C for 10 minutes. Thereafter, the reflection density was measured at 546 nm from the side of the support layer. The results are shown in Figs. 27 and 28.

Table 2

Buffers Used

Compound $A_1$: bis(2-hydroxyethyl)iminotris-(hydroxymethyl)methane
$(HOCH_2)_3CN(CH_2CH_2OH)_2$

Compound $B_1$: tris(hydroxymethyl)aminomethane
$(HOCH_2)_3CNH_2$

Compound $C_1$: sodium dihydrogen phosphate-disodium hydrogen phosphate

Compound $D_1$: N-tris(hydroxymethyl)methyl-2-aminoethane sulfonic acid
$(OHCH_2)_3CNHCH_2CH_2SO_3H$

Compound $E_1$: 3-[N-tris(hydroxymethyl)methylamino]-2-hydroxypropane sulfonic acid

Compound $F_1$: 3-[N,N-bis(2-hydroxyethyl)amino]-2-hydroxypropane sulfonic acid
$(OHCH_2CH_2)_2NCH_2CH(OH)CH_2SO_3H$

Among the compounds summerized in Table 2, Compound $A_1$ is an especially preferred buffering compound, Compounds $B_1$ and $C_1$ are conventional buffering compounds widely used in this field and Compounds $D_1$, $E_1$ and $F_1$ are analogues of Compound $A_1$. All of the Compounds $A_1$, $D_1$, $E_1$ and $F_1$ were commercially available from Dojindo Laboratories.

It can be seen from the above-described results that by employing the preferred buffer $A_1$, better results can be obtained than in the case of using the conventional bufferring compounds $B_1$ and $C_1$. Further, if the Compound $A_1$ is employed, better results can be obtained than in the case where the analogue compounds $D_1$, $E_1$ and $F_1$ are employed. Therefore, if a Good's buffering compound having a specific structure is employed, especially prominent results can be obtained.

Example 11

The same procedure as in Example 5 was repeated except that the buffers employed were those summarized in Table 3 below. The results are shown in Figs. 29 and 30.

Example 12

The same procedure as in Example 6 was repeated except that the buffers employed were those summerized in Table 3 below. The results are shown in Figs. 31 and 32.

Example 13

The same procedure as in Example 7 was repeated except that the buffers employed were those summerized in Table 3 below. The results are shown in Figs. 33 and 34.

Example 14

The same procedure as in Example 8 was repeated except that the buffers employed were those summerized in Table 3 below. The results are shown in Figs. 35 and 36.

Example 15

The same procedure as in Example 9 was repeated except that the buffers employed were those summerized in Table 3 below. The results are shown in Figs. 37 and 38.

Example 16

The same procedure as in Example 10 was repeated except that the buffers employed were those summerized in Table 3 below. The results are shown in Figs. 39 and 40.

Table 3

Compound $A_2$: piperazine-N,N'-bis(2-ethanesulfonic acid)

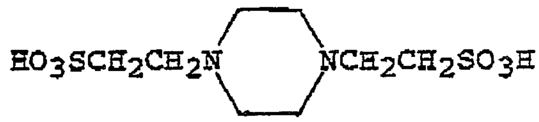

Compound $B_2$: N-2-hydroxyethylpiperazine-N'-2-ethane sulfonic acid

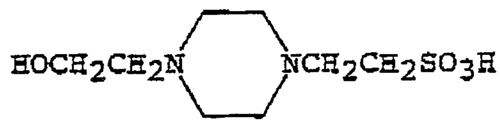

Compound $C_2$: tris(hydroxymethyl)aminomethane

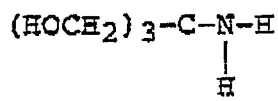

Compound $D_2$: sodium dihydrogen phosphate-disodium hydrogen phosphate

Compound $E_2$: 3-[N-tris(hydroxymethyl)methylamino]-2-hydroxypropane sulfonic acid

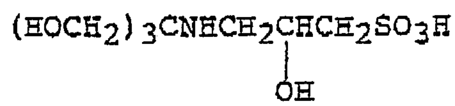

Compound $F_2$: 3-[N,N-bis(2-hydroxyethyl)amino]-2-hydroxypropane sulfonic acid

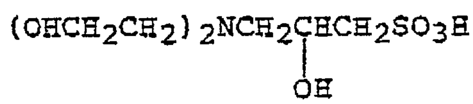

Among the compounds summerized in Table 3, Compound $A_2$ and $B_2$ are especially preferred buffering compounds, Compounds $C_2$ and $D_2$ are conventional buffering compounds widely used in this field and Compounds $E_2$ and $F_2$ are analogues of Compound $A_2$ or $B_2$. All of the Compounds $A_2$, $B_2$, $E_2$ and $F_2$ were commercially available from Dojindo Laboratories.

It can be seen from the above-described results that by employing the preferred buffer $A_2$ or $B_2$ better results can be obtained than in the case of using the conventional bufferring compounds $C_2$ and $D_2$. Further, if the Compound $A_2$ or $B_2$ is employed, better results can be obtained than in the case where the analogue compounds $E_2$ and $F_2$ are employed. Therefore, if a Good's buffering compound having a specific structure is employed, especially prominent results can be obtained.

Example 17

17-(1) Preparation of Epoxy Group-Introduced Avicel

In a mixture of 250 ml of butanedioldiglycidyl ether (manufactured by Aldorich) and 250 ml of 0.6 M aquous sodium hydroxide solution containing 2 mg/ml of sodiumborohydrate, 100 g of Avicel was suspended, and the suspension was shaken at 40° C for 5.5 hours. The thus treated Avicel was washed with 5 liters of pure water and dried.

17-(2) Preparation of Phenyl-β-D-thiogalactopyranoside-bound Avicel

In 60 ml of 0.1 M NaOH containing 1.5 g of phenyl-β-D-thiogalactopyranoside, 20 g of Avicel prepared in 17-(1) was added and the resulting mixture was then shaken at 45° C for 16 hours. After completion of the reaction, the Avicel was removed by filtration and was successively washed with water, 2.5 wt% glucose solution, water, 0.5 M NaCl-containing 0.1 M acetic acid buffer (pH 4.0), and water, and dried.

17-(3) Preparation of 5-aminoisophthalic acid-bound Avicel

In 110 ml of 2N NaOH, 20 g of 5-aminoisophthalic acid was dissolved and 30 g of the Avicel prepared in 17-(1) was added to the solution. The resulting mixture was shaken at 40° C for 20 hours. After completion of the reaction, the Avicel was removed by filtration, and successively washed with 0.2 N NaOH, water, 0.1 M acetic acid buffer (pH 4.0), water, 0.5 M NaCl-containing 0.1 M sodium hydrogen carbonate and water, followed by drying.

17-(4) Preparation of Avidin-bound Avicel and Goat Anti-human IgG Antibody-bound Avicel

In 1800 ml of 2.5 M phosphate buffer (pH 12.0), 90 g of Avicel was suspended and 45.0 g of cyanogen bromide dissolved in 550 ml of distilled water was added thereto while cooling the suspension in iced water. After 20 minutes, the Avicel was removed by filtration and was sufficiently washed with water. The thus treated 90 g of Avicel was suspended in 900 ml of 0.1 M sodium hydrogen carbonate containing 270 mg of avidin (Oriental Yeast Co., Ltd.) or 50 mg of goat anti-human IgG antibody (Cappel), and the resulting suspension was stirred at 4° C for 20 hours. The thus treated Avicel was removed and alternately washed with distilled water, 0.1 M aqueous sodium hydrogen carbonate solution containing 0.15 M sodium chloride and 0.1 M sodium acetate buffer (pH 4.1) containing 0.15 M sodium chloride, and was suspended in 1200 ml of 1 M tris-HCl buffer (pH 8.5). The suspension was stirred for 20 hours at room temperature to block the non-reacted reactive groups. The thus treated Avicel was removed by filtration, washed with water and lyophilized to obtain avidin-bound Avicel or goat anti-human IgG antibody-bound Avicel.

17-(5) Preparation of Rabbit Anti-human IgG Labelled with Glutamic Acid Dehydrogenase

To 0.2 ml of 0.1 M phosphate buffer (pH 6.8) containing 1.25 wt% of glutaraldehyde, 10 mg of glutamic acid dehydrogenase (EC1.4.l.4, originated from Proteus sp.) was added and the resulting mixture was gently stirred for 10 hours to allow the reaction. The resulting mixture was passed through Sephadex G-25 column (1.0 x 55 cm) preliminarily equilibrated with 0.15 M sodium chloride and the fraction of glutaraldehyde-activated glutamic acid dehydrogenase was collected. To the thus obtained 1.0 ml of the fraction, 1.0 ml of 0.15 M sodium chloride solution containing 5 mg of rabbit anti-human IgG (Cappel) and 0.1 ml of 1 M sodium carbonate buffer (pH 9.5) were added and the resulting mixture was allowed to react for 24 hours at 4° C. To this solution, 0.1 ml of 0.2 M lysine was added and the resultant was allowed to react for another two hours at 4° C. The resultant was passed through Ultrogel AcA-34 column (1.5 x 100 cm) (commercially available from Reactifs IBF-Societe Chimique Pointet-Girard (France), preliminarily equilibrated with 0.01 M phosphate buffer (pH 7.6) containing 0.15 M sodium chloride to obtain rabbit anti-human IgG labelled with glutamic acid dehydrogenase.

17-(5) Preparation of Immunoassay Element

The coating solutions (9) - (17) having the following compositions were prepared.

| Composition of coating solution (9) | |
|---|---|
| p-aminophenylmercuric acetate-bound Avicel | 14.0 g |
| Triton X-100 | 1.4 g |
| Polyvinylpyrrolidone (Wako Pure Chemicals) | 1.2 g |
| n-butanol | 34.0 g |

| Composition of coating solution (10) | |
|---|---|
| Phenyl-β-D-thiogalactosidepyranoside-bound Avicel | 14.0 g |
| Triton X-100 | 1.4 g |
| Polyvinylpyrrolidone (Wako Pure Chemicals) | 1.2 g |
| n-butanol | 34.0 g |

| Composition of coating solution (11) | |
|---|---|
| 5-aminoisophthalic acid-bound Avicel | 14.0 g |
| Triton X-100 | 1.4 g |
| Polyvinylpyrrolidone (Wako Pure Chemicals) | 1.2 g |
| n-butanol | 34.0 g |

| Composition of coating solution (12) | |
|---|---|
| Avidin-bound Avicel | 14.0 g |
| Triton X-100 | 1.4 g |
| Polyvinylpyrrolidone (Wako Pure Chemicals) | 1.2 g |
| 5-bromo-4-chloro-3-indolyl-β-D-galactopyranoside (Boehringer) | 0.9 g |
| NeoTB (Dojindo Laboratories) | 0.35 g |
| n-butanol | 34.0 g |

| Composition of coating solution (13) | |
|---|---|
| Avidin-bound Avicel | 14.0 g |
| Triton X-100 | 1.4 g |
| Polyvinylpyrrolidone (Wako Pure Chemicals) | 1.2 g |
| Chlorophenolred-β-D-galactopyranoside (CPRG) (Boehringer-Mannheim) | 0.90 g |
| n-butanol | 34.0 g |

| Composition of coating solution (14) | |
|---|---|
| Avidin-bound Avicel | 14.0 g |
| Polyvinylpyrrolidone (Wako Pure Chemicals) | 1.2 g |
| o-nitrophenyl-β-D-galactopyranoside (Merk) | 0.90 g |
| n-butanol | 34.0 g |

| Composition of coating solution (15) | |
|---|---|
| Avidin-bound Avicel | 14.0 g |
| Glutamic Acid | 0.30 g |
| 1-methoxy-5-methylphenazinium methylsulfate | 10 mg |
| Nicotinamideadeninedinucleotide phosphate (oxidized form) | 40 mg |
| NeoTB (Dojindo Laboratories) | 0.35 g |
| Triton X-100 | 1.4 g |
| Polyvinylpyrrolidone (Wako Pure Chemicals) | 1.2 g |
| n-butanol | 34.0 g |

| Composition of coating solution (16) | |
|---|---|
| Cellulose powder D (Toyo Filter Pater Co., Ltd.) | 40 mg |
| Triton X-100 | 3.0 g |
| Polyvinylpyrrolidone | 1.4 g |
| n-butanol | 80.0 g |

| Composition of coating solution (17) | |
|---|---|
| Goat anti-human IgG-bound Avicel | 14.0 g |
| Triton X-100 | 1.4 g |
| Polyvinylpyrrolidone (Wako Pure Chemicals) | 1.2 g |
| 5-bromo-4-chloro-3-indolyl-$\beta$-D-galactopyranoside (Boehringer) | 0.90 g |
| NeoTB | 0.35 g |
| n-butanol | 34.0 g |

The above coating solutions were coated on a transparent polyethyleneterephthalate film with a thickness of 180 $\mu$m in the order mentioned below in Table 4.

Table 4

| Element | First Solution | Second Solution | Third Solution |
|---|---|---|---|
| A | (9) | (12) | (16) |
| B | (9) | (13) | (16) |
| C | (9) | (14) | (16) |
| D | (10) | (12) | (16) |
| E | (10) | (13) | (16) |
| F | (10) | (14) | (16) |
| G | (9) | (15) | (16) |
| H | (11) | (15) | (16) |
| I | (9) | (17) | (16) |

Each sample was cut into pieces sizing 1.5 cm x 1.5 cm to obtain the assay elements.

17-(6) Measurement of Human IgG

The reagent solutions (a) - (e) having the following compositions were prepared.

Composition of reagent solution (a)

1 wt% bovine serum albumin
5 wt% collagen peptide A (bovine bone) (Funakoshi Pharmaceuticals)
0.3 M bis(2-hydroxyethyl)iminotris(hydroxymethyl)methane buffer (pH 7.2)
1 mM magnesium chloride
15 µg/ml rabbit anti-human IgG antibody labelled with β-D-galactosidase
100 µg/ml biotin-bound goat anti-human IgG antibody

Composition of reagent solution (b)

1 wt% of bovine serum albumin
5 wt% of collagen peptide A (bovine bone) (Funakoshi Pharmaceuticals)
0.3 M sodium phosphate buffer (pH 7.2)
1 mM magnesium chloride
15 µg/ml rabbit anti-human IgG antibody labelled with β-D-galactosidase
100 ug/ml biotin-bound goat anti-human IgG antibody

Composition of reagent solution (c)

1 wt% bovine serum albumin
5 wt% collagen peptide A (bovine bone) (Funakoshi Pharmaceuticals)
0.3 M N-2-hydroxyethylpiperazine-N′-2-hydroxypropane-3-sulfonic acid buffer (pH 8.5)
1 mM magnesium chloride
15 µg/ml rabbit anti-human IgG antibody labelled with glutamic acid dehydrogenase
100 µg/ml biotin-bound goat anti-human IgG antibody

Composition of Reagent Solution (d)

1 wt% bovine serum albumin
5 wt% collagen peptide A (bovine bone) (Funakoshi Pharmaceuticals)
0.3 M potassium phosphate buffer (pH 7.2)
1 mM magnesium chloride
15 µg/ml rabbit anti-human IgG antibody labelled with glutamic acid dehydrogenase
100 µg/ml biotin-bound goat anti-human IgG antibody

Composition of reagent solution (e)

1 wt% bovine serum albumin
5 wt% collagen peptide A (bovine bone) (Funakoshi Pharmaceuticals)
0.3 M bis(2-hydroxyethyl)iminotris(hydroxymethyl)methane buffer (pH 7.2)
1 mM magnesium chloride
15 µg/ml rabbit anti-human IgG antibody labelled with β-D-galactosidase

Further, human serum from which IgG was removed (manufactured by Miles Co., Ltd.) or the IgG-removed human serum to which 640 µg/ml of human IgG was added was used as the sample.

The reagent solution and the sample were mixed in the ratio of 4:1 (v/v) according to the combinations shown in Table 5. Immediately after mixing, the mixture was dropped on the assay element and the reflection density after incubation at 37° C for 20 minutes was measured.

Table 5

| No. | Assay Element | Reagent Solution | Measuring Wave Length (nm) | Dr 640 | Dr 0 | Dr640-Dr0 |
|---|---|---|---|---|---|---|
| 1 | A | a | 546 | 1.040 | 0.368 | 0.672 |
| 2 | I | e | 546 | 1.040 | 0.356 | 0.684 |
| 3 | B | a | 574 | 1.165 | 0.423 | 0.742 |
| 4 | A | b | 546 | 1.126 | 0.693 | 0.433 |
| 5 | B | b | 574 | 1.213 | 0.694 | 0.519 |
| 6 | C | a | 405 | 0.846 | 0.426 | 0.420 |
| 7 | C | b | 405 | 0.873 | 0.660 | 0.213 |
| 8 | D | a | 546 | 1.056 | 0.849 | 0.207 |
| 9 | E | a | 574 | 1.217 | 0.982 | 0.235 |
| 10 | F | a | 405 | 0.892 | 0.727 | 0.165 |
| 11 | G | c | 546 | 0.481 | 0.235 | 0.246 |
| 12 | G | d | 546 | 0.523 | 0.418 | 0.105 |
| 13 | H | c | 546 | 0.576 | 0.489 | 0.087 |

Dr 640: reflecting density obtained in measuring sample of IgG 640 µg/ml
Dr 0 : reflecting density obtained in measuring sample of IgG 0 µg/ml

The above-described results are summerized as follows:

1) The case wherein $Dr_{640}$ - $Dr_0$ is 0.670 or more (Nos. 1, 2 and 3)

This is the case in which β-D-galactosidase was used as the label and coloring substrate, buffer and inhibitor which are especially preferred in the present invention were employed.

2) The case wherein $Dr_{640}$ - $Dr_0$ is 0.520 - 0.420 (Nos. 4, 5 and 6)

This is the case in which β-D-galactosidase was used and two of the coloring substrate, buffer and inhibitor employed were those especially preferred in the present invention.

3) The case wherein $Dr_{640}$ - $Dr_0$ is less than 0.100 (No. 13)

This is the case in which the label was not β-D-galactosidase and the buffer and inhibitors were not those preferred in the present invention.

4) Others

Further, comparison of the cases in which β-D-galactosidase was employed and in which the other marker was employed in the same conditions is summerized in Table 6.

Table 6

| Experiment | $Dr_{640} - Dr_0$ | |
|---|---|---|
| No. 1 | 0.672 | label of the present invention was used |
| No. 2 | 0.654 | |
| No. 3 | 0.742 | |
| No. 6 | 0.420 | |
| No. 11 | 0.246 | comparative example |
| No. 4 | 0.433 | label of the present invention was used |
| No. 5 | 0.519 | |
| No. 7 | 0.213 | |
| No. 12 | 0.105 | comparative example |
| No. 8 | 0.207 | label of the present invention was used |
| No. 9 | 0.235 | |
| No. 10 | 0.165 | |
| No. 13 | 0.087 | comparative example |

Example 18

18-(1) Preparation of p-aminophenylmercuric Acetate-bound Avicel

P-aminophenylmercuric acetate-bound Avicel was prepared in the same manner as in Example 1-(1).

18-(2) Preparation of Biotin-bound Avicel

In 330 ml of 0.01 M phosphate buffer (pH 7.4) containing 0.15 M sodium chloride, 1.0 g of bovine serum albumin (fraction V, manufactured by Arma Co., Ltd., U.S.) was dissolved. To this solution, 10.4 mg of biotin-N-hydroxy succinimide ester (manufactured by Boehringer) in 3.0 ml of dimethylformamide was added and the resulting mixture was allowed to react at room temperature for 2.5 hours. The resulting reaction mixture was sufficiently dialyzed against the same buffer and was lyophilized to obtain bovine serum albumin to which biotin was bound. In 1800 ml of 2.5 M phosphate buffer (pH 12.0), 90 g of Avicel was suspended, and 45.0 g of cyanogen bromide in 550 ml of distilled water was added thereto while cooling the suspension in iced water. After allowing to react for 20 minutes, the Avicel was removed by filtration and was sufficiently washed with water. Ninety grams of this Avicel was suspended in 900 ml of 0.1 M sodium hydrogen carbonate solution containing 500 mg of the bovine serum albumin to which biotin was bound and containing 0.15 M sodim chloride, and the suspension was stirred at 4° C for 20 hours. The thus treated Avicel was removed by filtration and was alternately washed with distilled water, 0.1 M aqueous sodium hydrogen carbonate solution containing 0.15 M sodium chloride, and 0.1 M sodium acetate buffer (pH 4.1) containing 0.15 M sodium chloride, and was suspended in 1200 ml of 1 M Tris-HCl buffer (pH 8.5). The resulting suspension was stirred at room temperature for 20 hours to block the non-reacted reactive groups. The Avicel was removed by filtration and sufficiently washed with water to obtain Avicel to which

biotin was bound. Then 80 g of the biotin-bound Avicel was suspended in 240 ml of pure water containing 10.0 g of bovine serum albumin and the resulting suspension was lyophilized to obtain biotin-bound Avicel containing bovine serum albumin.

18-(3) Preparation of β-galactosidase-labelled human IgG

β-galactosidase-labelled human IgG was prepared in the same manner as in Example 1-(3).

18-(4) Preparation of Biotin-bound Goat Anti-human IgG Antibody

Biotin-bound goat anti-human IgG antibody was prepared in the same manner as in Example 1-(4).

18-(5) Preparation of Immunoassay Element

The same operation as in Example 1-(5) was repeated except that the biotin-bound Avicel did not contain sucrose, to obtain an immunoassay element.

18-(6) Measurement of Human IgG

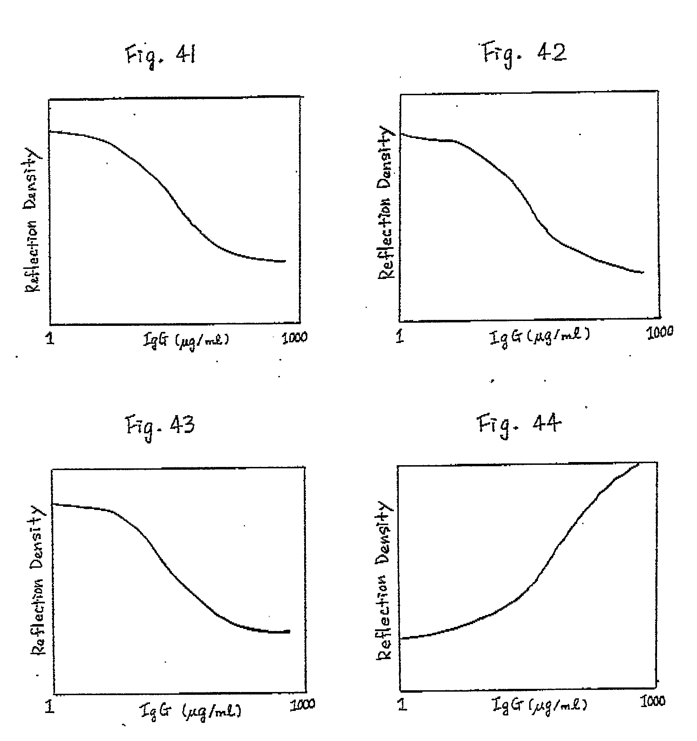

Human IgG in the samples were assayed in the same manner as in Example 1-(6) except that the mixture applied to the immunoassay element further contained avidin (100 ug/ml solution in Bistris (pH 7.2)-). The results are shown in Fig. 41.

Example 19

19-(1) Preparation of Biotin-bound Cellulose Fibers

The same procedure as in 18-(1) was repeated except that cellulose powder D was used in place of Avicel to prepare biotin-bound cellulose fibers.

19-(2) Preparation of Immunoassay Element

On a transparent polyethyleneterephthalate film with a thickness of 180 μm, a coating solution (18) with the following composition was applied and dried to form a gelatin layer.

| Composition of Coating Solution (18) | |
|---|---|
| Deionized Gelatin | 4.5 g |
| Triton X-100 | 0.10 g |
| 1,2-bis(vinylsulfonyl)ethane | 0.007 g |
| NeoTB | 0.23 g |
| Distilled Water | 40.5 g |

Then a dispersion with the same composition as the coating solutin (2) described in 1-(5) except that the dispersion does not contain NeoTB was applied on the gelatin layer and dried to form p-aminophenylmercuric acetate-bound Avicel layer.

On this layer, The coating solution (19) having the following composition and the coating solution (4) described in 1-(5) were successively applied and dried. The sample was then cut into pieces sizing 1.5 cm x 1.5 cm to obtain an immunoassay element.

| Composition of Coating Solution (19) | |
|---|---|
| Biotin-bound Cellulose Powder D | 30.0 g |
| Avidin | 2.5 mg |
| Triton X-100 | 3.0 g |
| Bovine Serum Albumin | 2.0 g |
| Polyvinylpyrrolidone | 1.5 g |
| n-butanol | 55.0 g |

19-(3) Measurement of Human IgG

The human IgG in samples were measured in the same manner as in 1-(6). The results are shown in Fig. 42.

Example 20

20-(1) Preparation of Immunoassay Element

On a polyethyleneterephthalate film with a thickness of 180 µm, the coating solution (5) described in 2-(2) was applied and dried to form a gelatin layer. Then a dispersion with the same composition as the coating solution (2) described in 1-(5) except not containing NeoTB was applied and dried to form p-aminophenylmercuric acetate-bound Avicel layer.

On this layer, a coating solution (20) with the following composition containing the human IgG labelled with β-D-galactosidase prepared in 1-(3), the biotin-bound goat anti-human IgG antibody prepared in 1-(4) and avidin was applied and dried. Further, on this layer, the coating solution (4) described in 1-(5) was applied and dried. The sample was then cut into pieces sizing 1.5 cm x 1.5 cm to obtain an immunoassay element.

| Composition of Coating Solution (20) | |
|---|---|
| Avidin-bound Avicel containing bovine serum albumin | 22.5 g |
| Triton X-100 | 2.50 g |
| Polyvinylpyrrolidone | 3.30 g |
| Human IgG labelled with β-D-galactosidase | 0.16mg |
| Biotin-bound goat anti-human IgG antibody | 0.60mg |
| Avidin | 1.5 mg |
| Bovine serum albumin | 150 mg |
| n-butanol | 55.0 g |

20-(2) Measurement of Human IgG

Human IgG (0 - 640 µg/ml) dissolved in 0.3 M Bristris-HCl buffer (pH 7.2) containing 1 mM magnesium chloride and 6 wt% of bovine serum albumin was dropped on the thus prepared immunoassay element and the element was incubated in sealed condition at 37° C for 10 minutes. Reflection density was measured at 546 nm from the side of the support layer. The results are shown in Fig. 43.

Example 21

21-(1) Preparation of Rabbit Anti-human IgG Antibody Labelled with β-D-galactosidase

In the same manner as in 1-(3) using 20 mg of rabbit anti-human IgG antibody (manufactured by Cappel), rabbit anti-human IgG antibody labelled with β-D-galactosidase was obtained.

21-(2) Measurement of Human IgG

Human IgG (0 - 640 μg/ml) dissolved in 0.3 M Bistris-HCl buffer (pH 7.2) containing 1 mM magnesium chloride and 6 wt% of bovine serum albumin, rabbit anti-human IgG antibody labelled with β-D-galactosidase (15 μg/ml solution in the same buffer), and biotin-bound goat anti-human IgG antibody (100 μg/ml solution in the same buffer) were mixed and 10 μl of the mixture was dropped on the element prepared in 19-(2). The element was incubated in sealed condition at 37° C for 10 minutes and the reflection density was measured from the side of the support layer at 546 nm. The results are shown in Fig. 44.

Although the invention was described based on the preferred specific examples thereof, it is apparent for those skilled in the art that various modifications may be made without departing from the scope and the spirit of the present invention.

## Claims

1. A method of assaying a target substance in a fluid sample utilizing a specific binding reaction between the target substance and a substance which specifically binds to the target substance, comprising the steps of:
reacting

a) the target substance;

b) the substance which specifically binds to the target substance, to which a biological active substance which does not bind to the target substance is attached, or to which a substance which specifically binds to a biological active substance which does not bind to the target substance is attached;

c) a labelled substance which is the target substance or an analogue thereof labelled with β-D-galactosidase, or which is a substance which specifically binds to the target substance, labelled with β-D-galactosidase,

d) a substance which specifically binds to the biological active substance and which does not bind to the target substance, or a biological active substance, which is fixed to a carrier, which carrier exists in a porous reaction layer of an assay element, and

e) a substance which specifically binds to β-D-galactosidase and which changes a signal originated from β-D-galactosidase, which is fixed to said carrier or another carrier which exists in a porous reaction layer of an assay element; and

measuring the change of the signal from β-D-galactosidase, thereby detecting the existence of the target substance or determining the amount of the target substance in the fluid sample.

2. The method of claim 1, wherein the β-D-galactosidase is originated from E. coli.

3. The method of claim 1, wherein the change of the signal originated from β-D-galactosidase is detected by measuring the activity of β-D-galactosidase by generating a color or fluorescent light utilizing a substance selected from the group consisting of 5-bromo-4-chloro-3-indolyl-β-D-galactoside, 5-bromo-2-naphtyl-β-D-galactoside, 5-carboxy-2-nitrophenyl-β-D-galactoside, isopropyl-β-D-thiogalactoside, 4-methylumbelliferyl-β-D-galactoside, o-nitrophenyl-β-D-galactoside, o-nitrophenyl-1-thio-β-D-galactoside, phenyl-β-D-galactoside, 3,6-dihydroxyfluoran-β-D-galactoside, 6-bromo-2-naphtyl-β-D-galactoside, 2-naphtyl-β-D-galactoside, fluorescein-di-β-D-galactoside; and measuring absorbance of the color or intensity of the fluorescent light.

4. The method of claim 1, wherein the change of the signal originated from β-D-galactosidase is detected by measuring the activity of β-D-galactosidase by generating a color utilizing indolyl-β-D-galactopyranoside derivative.

5. The method of claim 4, wherein the indolyl- β-D-galactopyranoside derivative is at least one selected from the group consisting of 3-indolyl-β-D-galactopyranoside, 5-bromo-3-indolyl-β-D-galactopyranoside and 5-bromo-4-chloro-3-indolyl-β-D-galactopyranoside.

6. The method of claim 3 or 4, wherein a tetrazolium salt is utilized as a coloring agent for the detection of the change of the absorbance.

7. The method of claim 1, wherein the change of the signal originated from β-D-galactosidase is detected by measuring the activity of β-D-galactosidase by generating a color utilizing chlorophenolred-β-D-galactopyranoside derivative.

8. The method of claim 1, wherein the biological active substance and the substance which specifically binds to the biological active substance is an antibody and the corresponding antigen, or biotin or a derivative thereof and avidin or a derivative thereof.

9. The method of claim 1, wherein the substance which changes the signal originated from β-D-galactosidase is an organic mercury compound.

10. The method of claim 1, wherein the substance which changes the signal originated from β-D-galactosidase is selected from the group consisting of an organic silver compound, galactostatin or a derivative thereof, p-aminophenyl-β-D-thiogalactopyranoside and o-mercuryphenyl-β-D-galactoside chloride.

11. The method of claim 8, wherein the organic mercury compound is an aryl mercury derivative.

12. The method of claim 11, wherein the reaction is conducted in the presence of a compound represented by the formula [I] or a derivative thereof:

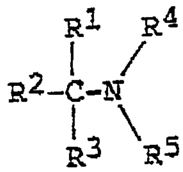

[I]

(wherein $R^1$, $R^2$, $R^3$, $R^4$ and $R^5$, the same or different, represent $C_1$ - $C_5$ alkyl group which has a hydroxide group or a sulfonyl group as a substituent on the main chain thereof).

13. The method of claim 12, wherein the compound represented by the formula [I] is bis(2-hydroxyethyl)iminotris(hydroxymethyl)methane.

14. The method of claim 13, wherein the reaction is conducted in the presence of a compound represented by the formula [II] or a salt thereof:

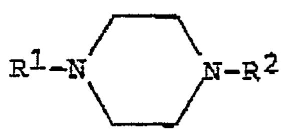

[II]

(wherein $R^1$ and $R^2$, the same or different, represent the same meaning as in formula [I]).

15. The method of claim 14, wherein the compound represented by the formula [II] is piperazine-N,N′-bis(2-ethanesulfonic acid) or N-2-hydroxyethylpiperazine-N′-2-ethanesulfonic acid.

16. An immunoassay element comprising:

a biological active substance which does not binds to a target substance in a fluid sample, or a substance which specifically binds to a biological active substance which does not binds to a target substance in a fluid sample, which is fixed to a carrier contained in a porous reaction layer of the immunoassay element; and

a substance which specifically binds to β-D-galactosidase and which changes a signal originated from β-D-galactosidase, which is fixed to said carrier or another carrier existing in the porous rection layer of the immunoassay element.

17. The immunoassay element of claim 16, wherein the β-D-galactosidase is originated from E. coli.

18. The immunoassay element of claim 15, further comprising a substance selected from the group consisting of 5-bromo-4-chloro-3-indolyl-β-D-galactoside, 5-bromo-2-naphtyl-β-D-galactoside, 5-carboxy-2-nitrophenyl-β-D-galactoside, isopropyl-β-D-thiogalactoside, 4-methylumbelliferyl-β-D-galactoside, o-nitrophenyl-β-D-galactoside, o-nitrophenyl-1-thio-β-D-galactoside, phenyl-β-D-galactoside, 3,6-dihydroxyfluoran-β-D-galactoside, 6-bromo-2-naphtyl-β-D-galactoside, 2-naphytyl-β-D-galactoside, fluorescein-di-β-D-galactoside for generating a color or fluorescent light for the detection of the change of the signal originated from β-D-galactosidase.

19. The immunoassay element of claim 16, further comprising indolyl-β-D-galactopyranoside derivative for generating a color or fluorescent light for the detection of the change of the signal originated from β-D-galactosidase.

20. The immunoassay element of claim 19, wherein the indolyl-β-D-galactopyranoside derivative is at least one selected from the group consisting of 3-indolyl-β-D-galactopyranoside, 5-bromo-3-indolyl-β-D-galactopyranoside and 5-bromo-4-chloro-3-indolyl-β-D-galactopyranoside.

21. The immunoassay element of claim 18 or 19, further comprising a tetrazolium salt is utilized as a coloring agent for the detection of the change of the absorbance.

22. The immunoassay element of claim 16, further comprising chlorophenolred-β-D-galactopyranoside derivative for generating a color or fluorescent light for the detection of the change of the signal originated from β-D-galactosidase.

23. The immunoassay element of claim 16, wherein the biological active substance and the substance which specifically binds to the biological active substance is an antibody and the corresponding antigen, or biotin or a derivative thereof and avidin or a derivative thereof.

24. The immunoassay element of claim 16, wherein the substance which changes the signal originated from β-D-galactosidase is an organic mercury compound.

25. The immunoassay element of claim 16, wherein the substance which changes the signal originated from β-D-galactosidase is selected from the group consisting of an organic silver compound, galactostatin or a derivative thereof, p-aminophenyl-β-D-thiogalactopyranoside and o-mercuryphenyl-β-D-galactoside chloride.

26. The immunoassay element of claim 24, wherein the organic mercury compound is an aryl mercury derivative.

27. The immunoassay element of claim 24, further comprising a compound represented by the formula [I] or a derivative thereof:

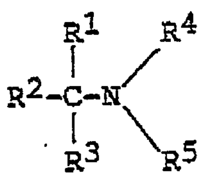

[I]

(wherein $R^1$, $R^2$, $R^3$, $R^4$ and $R^5$, the same or different, represent $C_1$ - $C_5$ alkyl group which has a hydroxide group or a sulfonyl group as a substituent on the main chain thereof).

28. The immunoassay element of claim 27, wherein the compound represented by the formula [I] is bis-(2-hydroxyethyl)iminotris(hydroxymethyl)methane.

29. The method of claim 24, further comprising a compound represented by the formula [II] or a salt thereof:

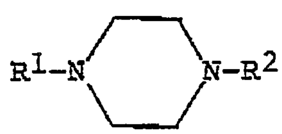

[II]

(wherein $R^1$ and $R^2$, the same or different, represent the same meaning as in formula [I]).

30. The immunoassay element of claim 29, wherein the compound represented by the formula [II] is piperadine-N,N′-bis(2-ethanesulfonic acid) or N-2-hydroxyethylpiperadine-N′-2-ethanesulfonic acid.

31. The immunoassay element of claim 16, wherein the porous reaction layer is made of particles with a particle size of 1 - 350 µm, or a fabric with a mesh size of 40 - 400 mesh.

32. A method of assaying a target substance in a fluid sample utilizing a specific binding reaction between the target substance and a substance which specifically binds to the target substance, comprising the steps of:

reacting

a) the target substance,

b) a labelled substance which is the target substance or an analogue thereof labelled with β-D-galactosidase, or a labelled substance which is a substance which specifically binds to the target substance, labelled with β-D-galactosidase,

c) the substance which specifically binds to the target substance, which is fixed to a carrier contained in a porous reaction layer of an immunoassay element, and

d) a substance which specifically binds to β-D-galactosidase and which changes a signal originated from β-D-galactosidase, which is fixed to said carrier or another carrier which exists in a porous reaction layer of an assay element; and

measuring the change of the signal from β-D-galactosidase, thereby detecting the existence of the target substance or determining the amount of the target substance in the fluid sample.

33. The method of claim 32, wherein the β-D-galactosidase is originated from E. coli.

34. The method of claim 32, wherein the change of the signal originated from β-D-galactosidase is detected by measuring the activity of β-D-galactosidase by generating a color or fluorescent light utilizing a substance selected from the group consisting of 5-bromo-4-chloro-3-indolyl-β-D-galactoside, 5-bromo-2-naphtyl-β-D-galactoside, 5-carboxy-2-nitrophenyl-β-D-galactoside, isopropyl-β-D-thiogalactoside, 4-methylumbelliferyl-β-D-galactoside, o-nitrophenyl-β-D-galactoside, o-nitrophenyl-1-thio-β-D-galactoside, phenyl-β-D-galactoside, 3,6-dihydroxyfluoran-β-D-galactoside, 6-bromo-2-naphtyl-β-D-galactoside, 2-naphtyl-β-D-galactoside, fluorescein-di-β-D-galactoside; and measuring absorbance of the color or intensity of the fluorescent light.

35. The method of claim 32, wherein the change of the signal originated from β-D-galactosidase is detected by measuring the activity of β-D-galactosidase by generating a color utilizing indolyl-β-D-galactopyranoside derivative.

36. The method of claim 35, wherein the indolyl-β-D-galactopyranoside derivative is at least one selected from the group consisting of 3-indolyl-β-D-galactopyranoside, 5-bromo-3-indolyl-β-D-galactopyranoside and 5-bromo-4-chloro-3-indolyl-β-D-galactopyranoside.

37. The method of claim 34 or 35, wherein a tetrazolium salt is utilized as a coloring agent for the detection of the change of the absorbance.

38. The method of claim 32, wherein the change of the signal originated from β-D-galactosidase is detected by measuring the activity of β-D-galactosidase by generating a color utilizing chlorophenolred-β-D-galactopyranoside derivative.

39. The method of claim 32, wherein the substance which changes the signal originated from β-D-galactosidase is an organic mercury compound.

40. The method of claim 32, wherein the substance which changes the signal originated from β-D-galactosidase is selected from the group consisting of an organic silver compound, galactostatin or a derivative thereof, p-aminophenyl-β-D-thiogalactopyranoside and o-mercuryphenyl-β-D-galactoside chloride.

41. The method of claim 39, wherein the organic mercury compound is an aryl mercury derivative.

42. The method of claim 41, wherein the reaction is conducted in the presence of a compound represented by the formula [I] or a derivative thereof:

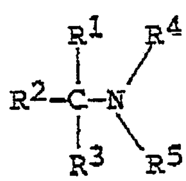

[I]

(wherein $R^1$, $R^2$, $R^3$, $R^4$ and $R^5$, the same or different, represent $C_1$ - $C_5$ alkyl group which has a hydroxide group or a sulfonyl group as a substituent on the main chain thereof).

43. The method of claim 42, wherein the compound represented by the formula [I] is bis(2-hydroxyethyl)iminotris(hydroxymethyl)methane.

44. The method of claim 43, wherein the reaction is conducted in the presence of a compound represented by the formula [II] or a salt thereof:

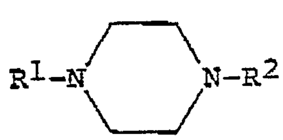

[II]

(wherein $R^1$ and $R^2$, the same or different, represent the same meaning as in formula [I]).

45. The method of claim 44, wherein the compound represented by the formula [II] is piperazine-N,N′-bis(2-ethanesulfonic acid) or N-2-hydroxyethylpiperazine-N′-2-ethanesulfonic acid.

46. An immunoassay element comprising:
a substance which specifically binds to a target substance in a fluid sample, which is fixed to a carrier existing in a porous reaction layer of the immunoassay element; and
a substance which specifically binds to β-D-galactosidase and which changes a signal oroginated from β-D-galactosidase, which is fixed to said carrier or another carrier existing in a porous reaction layer of the immunoassay element.

47. The immunoassay element of claim 46, wherein the β-D-galactosidase is originated from E. coli.

48. The immunoassay element of claim 46, further comprising a substance selected from the group consisting of 5-bromo-4-chloro-3-indolyl-β-D-galactoside, 5-bromo-2-naphtyl-β-D-galactoside, 5-carboxy-2-nitrophenyl-β-D-galactoside, isopropyl-β-D-thiogalactoside, 4-methylumbelliferyl-β-D-galactoside, o-nitrophenyl-β-D-galactoside, o-nitrophenyl-1-thio-β-D-galactoside, phenyl-β-D-galactoside, 3,6-dihydroxyfluoran-β-D-galactoside, 6-bromo-2-naphtyl-β-D-galactoside, 2-naphytyl-β-D-galactoside, fluorescein-di-β-D-galactoside for generating a color or fluorescent light for the detection of the change of the signal originated from β-D-galactosidase.

49. The immunoassay element of claim 46, further comprising indolyl-β-D-galactopyranoside derivative for generating a color or fluorescent light for the detection of the change of the signal originated from β-D-galactosidase.

50. The immunoassay element of claim 49, wherein the indolyl-β-D-galactopyranoside derivative is at least one selected from the group consisting of 3-indolyl-β-D-galactopyranoside, 5-bromo-3-indolyl-β-D-galactopyranoside and 5-bromo-4-chloro-3-indolyl-β-D-galactopyranoside.

51. The immunoassay element of claim 48 or 49, further comprising a tetrazolium salt is utilized as a coloring agent for the detection of the change of the absorbance.

52. The immunoassay element of claim 46, further comprising chlorophenolred-β-D-galactopyranoside derivative for generating a color or fluorescent light for the detection of the change of the signal originated from β-D-galactosidase.

53. The immunoassay element of claim 46, wherein the substance which changes the signal originated from β-D-galactosidase is an organic mercury compound.

54. The immunoassay element of claim 46, wherein the substance which changes the signal originated from β-D-galactosidase is selected from the group consisting of an organic silver compound, galactostatin or a derivative thereof, p-aminophenyl-β-D-thiogalactopyranoside and o-mercuryphenyl-β-D-galactoside chloride.

55. The immunoassay element of claim 53, wherein the organic mercury compound is an aryl mercury derivative.

56. The immunoassay element of claim 53, further comprising a compound represented by the formula [I] or a derivative thereof:

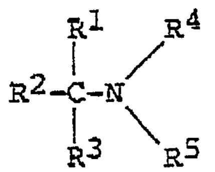

[I]

(wherein $R^1$, $R^2$, $R^3$, $R^4$ and $R^5$, the same or different, represent $C_1$ - $C_5$ alkyl group which has a hydroxide group or a sulfonyl group as a substituent on the main chain thereof).

57. The immunoassay element of claim 56, wherein the compound represented by the formula [I] is bis-(2-hydroxyethyl)iminotris(hydroxymethyl)methane.

58. The method of claim 53, further comprising a compound represented by the formula [II] or a salt thereof:

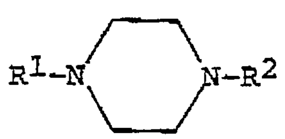

[II]

(wherein $R^1$ and $R^2$, the same or different, represent the same meaning as in formula [I]).

59. The immunoassay element of claim 58, wherein the compound represented by the formula [II] is piperadine-N,N′-bis(2-ethanesulfonic acid) or N-2-hydroxyethylpiperazine-N′-2-ethanesulfonic acid.

60. The immunoassay element of claim 46, wherein the porous reaction layer is made of particles with a particle size of 1 - 350 μm, or a fabric with a mesh size of 40 - 400 mesh.

FIG. 1

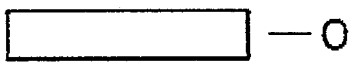


FIG. 2

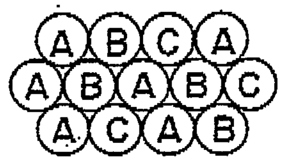


FIG. 3

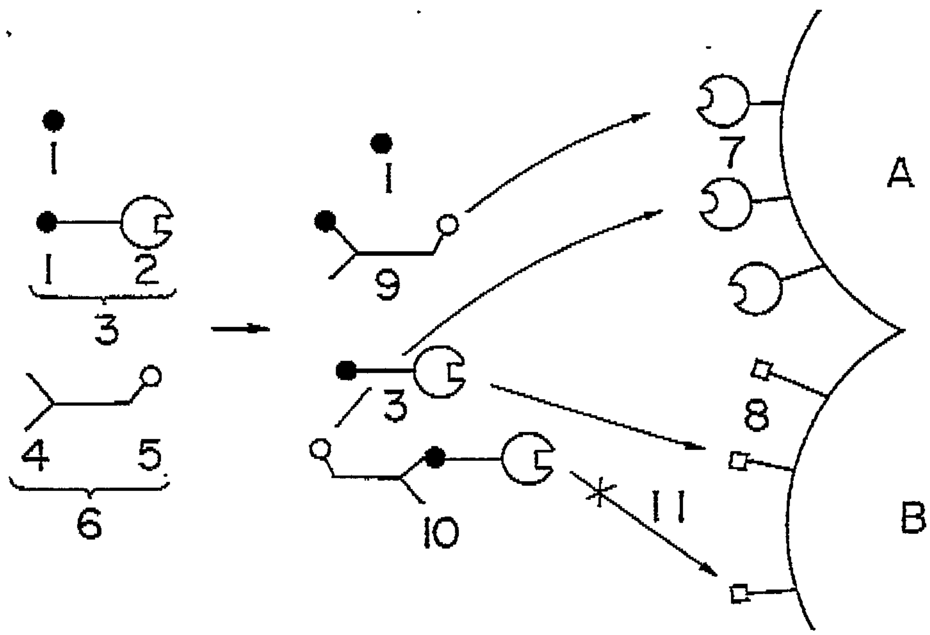


FIG. 4

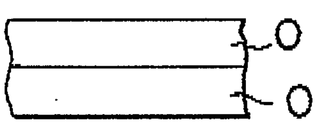


FIG. 5

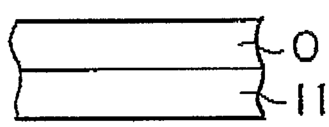

Fig. 3'

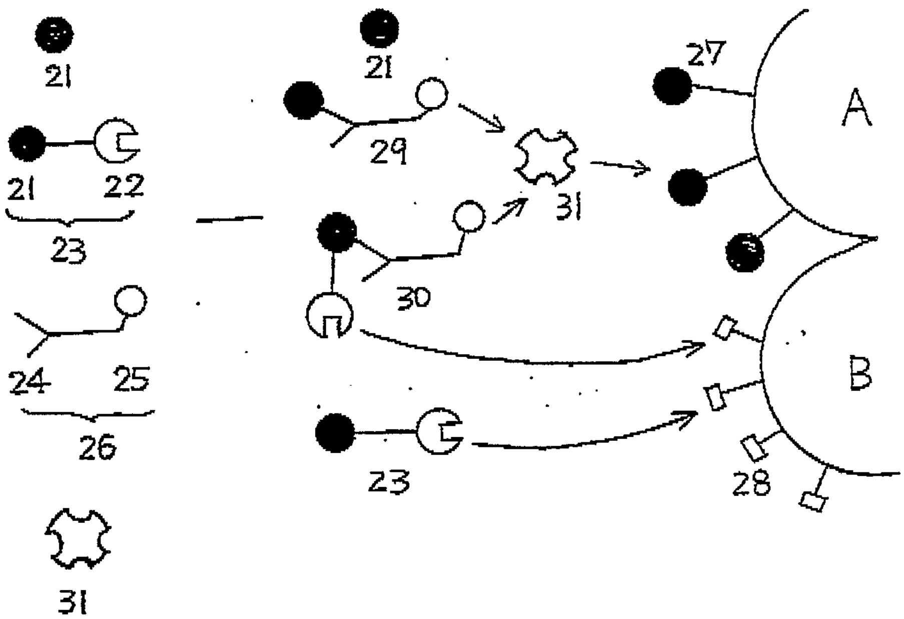

FIG. 6

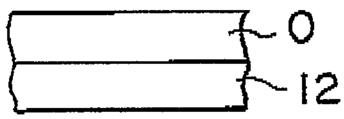


FIG. 7

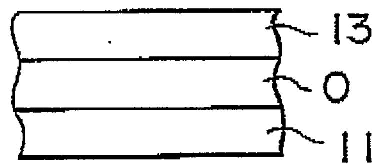


FIG. 8

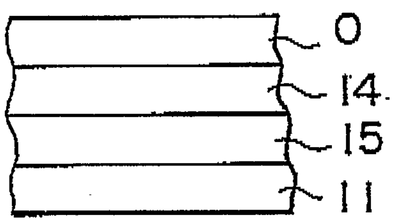


FIG. 9

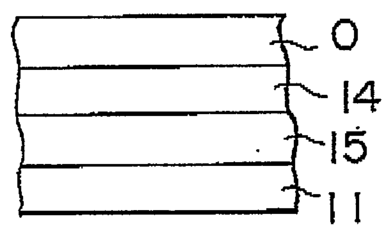


FIG. 10

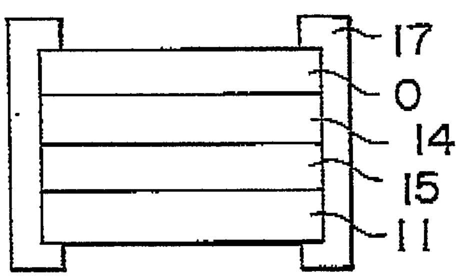


FIG. 11

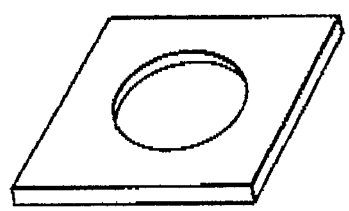

FIG. 12

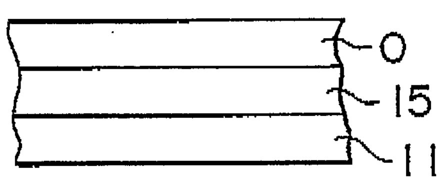

FIG. 13

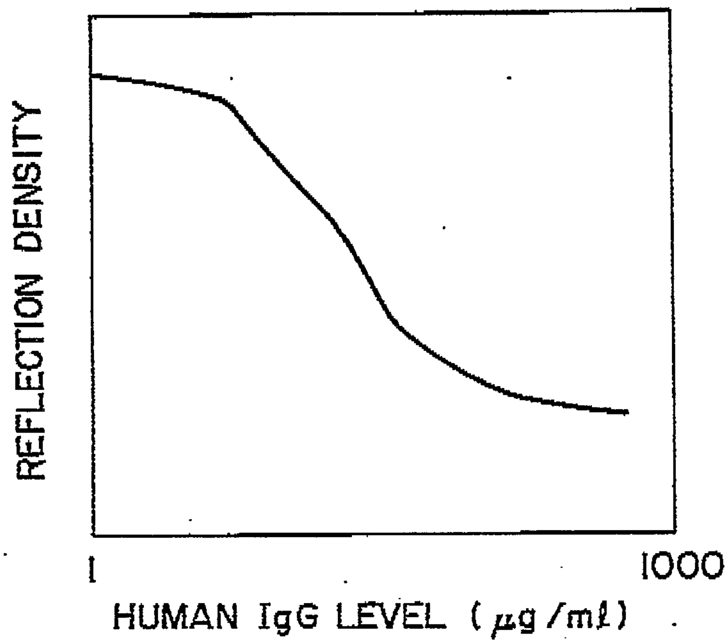
REFLECTION DENSITY
1
1000
HUMAN IgG LEVEL (μg/mℓ)

FIG. 14

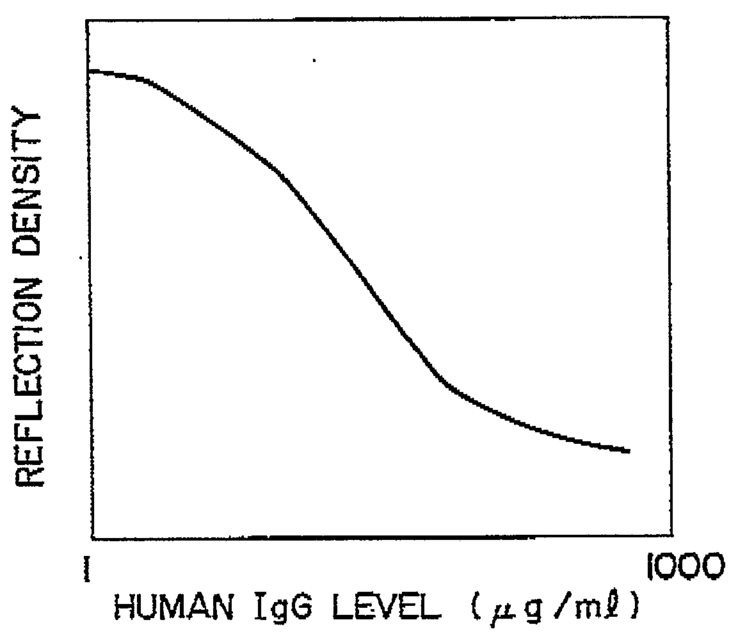
REFLECTION DENSITY
1
1000
HUMAN IgG LEVEL (μg/mℓ)

FIG. 15

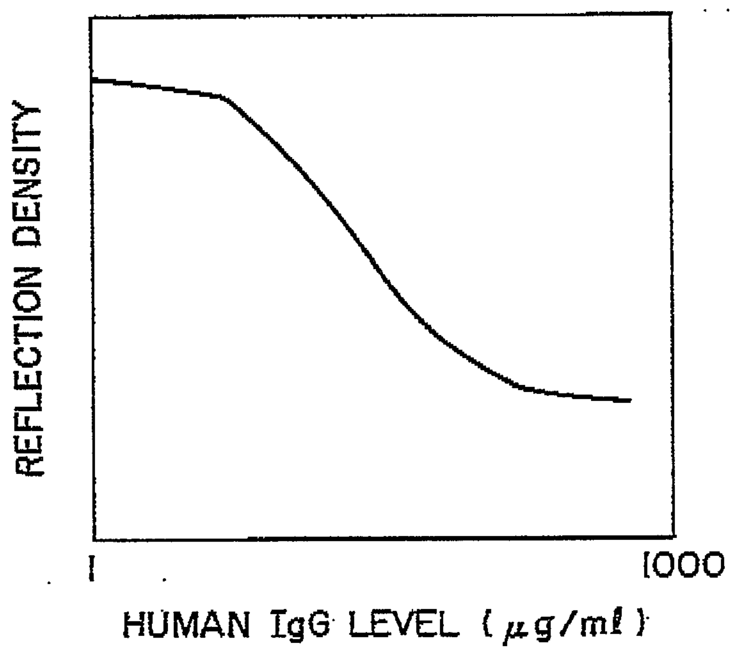
REFLECTION DENSITY
1
1000
HUMAN IgG LEVEL (μg/ml)

FIG. 16

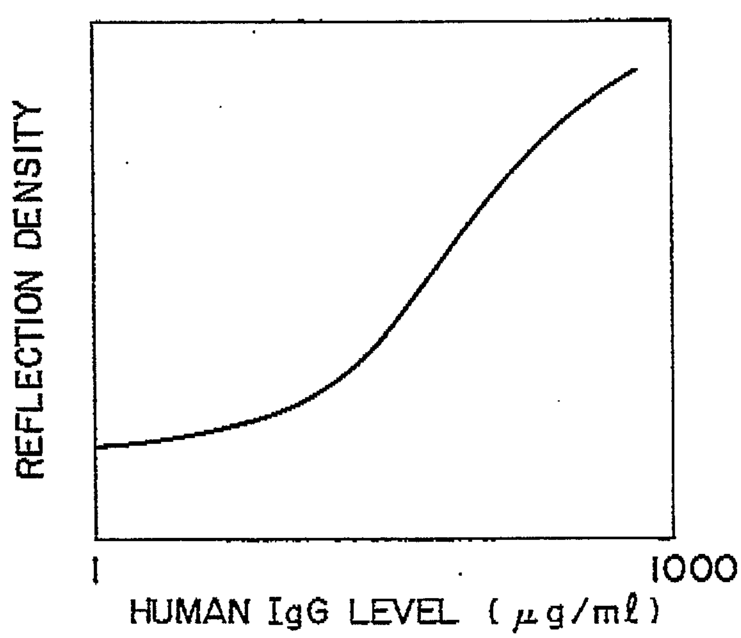
REFLECTION DENSITY
1
1000
HUMAN IgG LEVEL (μg/ml)

# FIG. 17

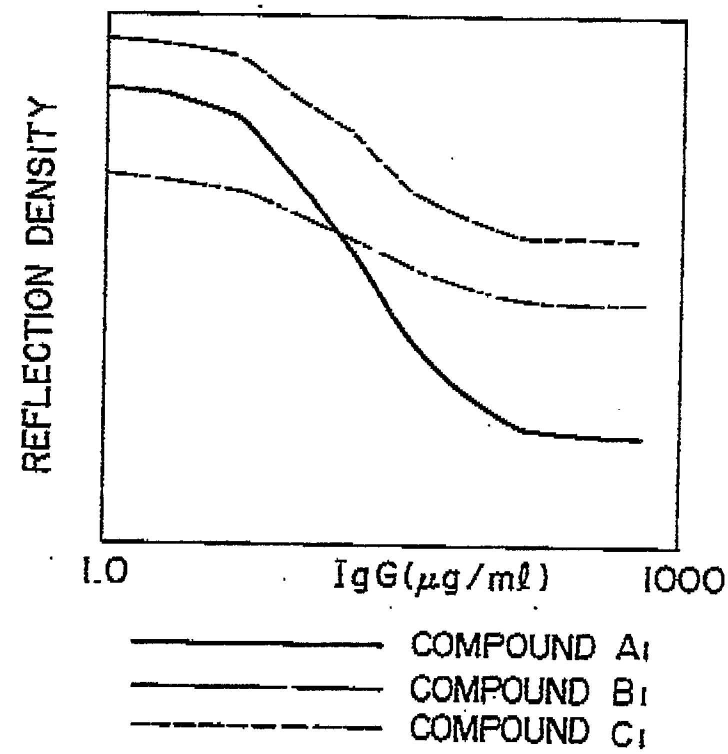
REFLECTION DENSITY
1.0
IgG (μg/mℓ)
1000
COMPOUND A1
COMPOUND B1
COMPOUND C1

# FIG. 18

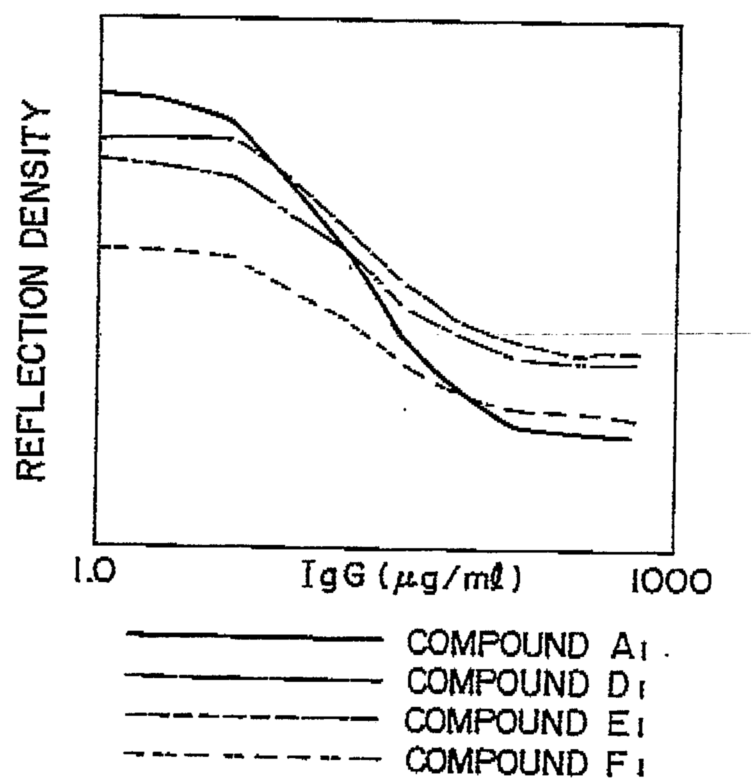
REFLECTION DENSITY
1.0
IgG (μg/mℓ)
1000
COMPOUND A1
COMPOUND D1
COMPOUND E1
COMPOUND F1

FIG. 19

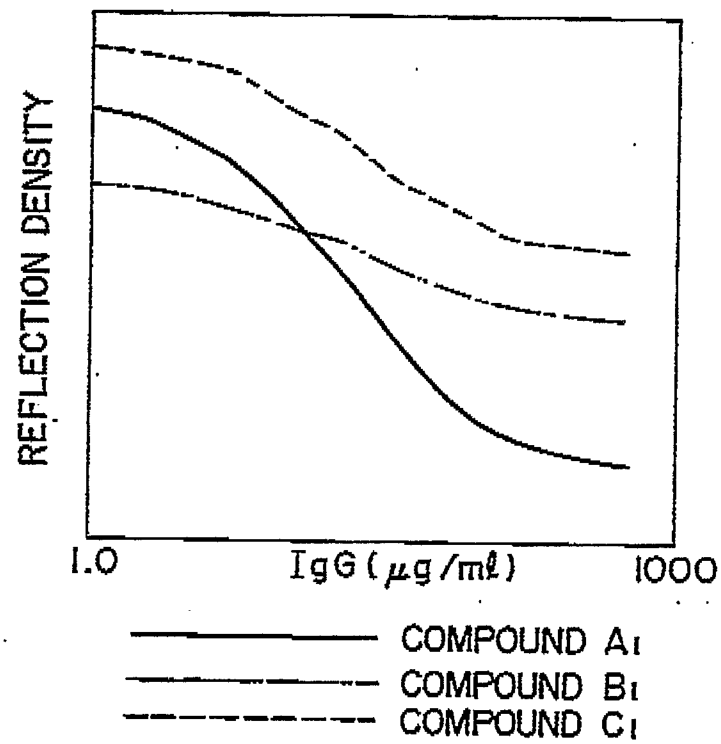
REFLECTION DENSITY
1.0
IgG (μg/mℓ)
1000
COMPOUND A1
COMPOUND B1
COMPOUND C1

FIG. 20

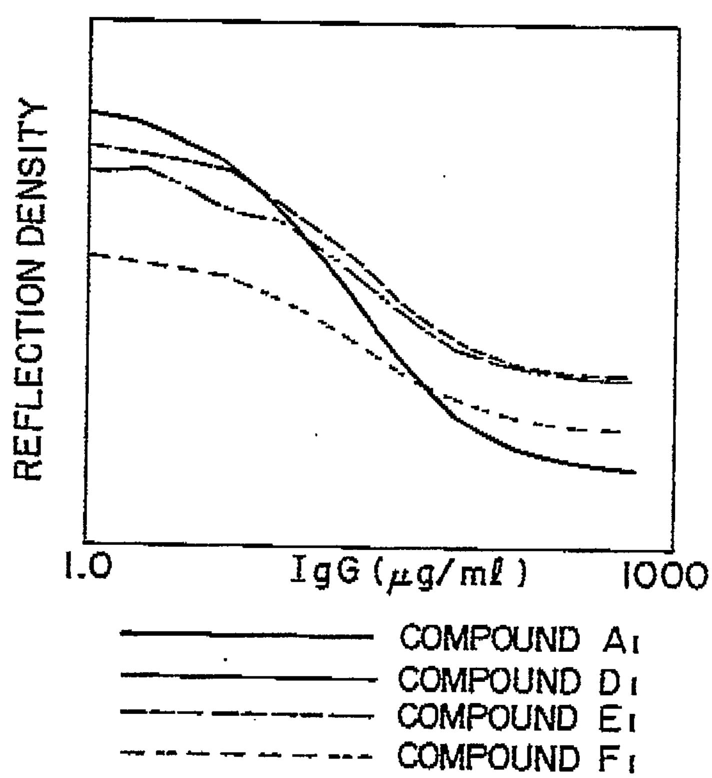
REFLECTION DENSITY
1.0
IgG (μg/mℓ)
1000
COMPOUND A1
COMPOUND D1
COMPOUND E1
COMPOUND F1

# FIG. 21

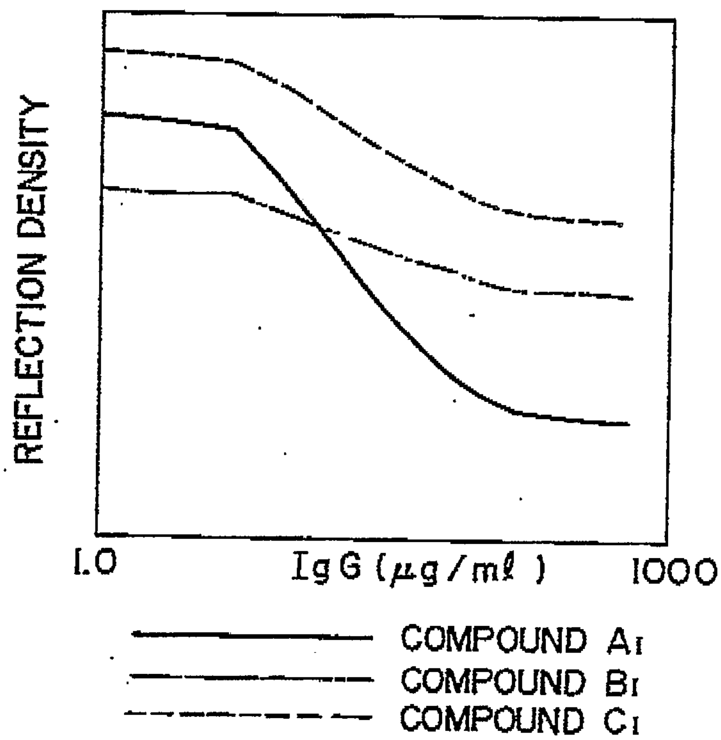
REFLECTION DENSITY
1.0
IgG (μg/mℓ)
1000
COMPOUND A1
COMPOUND B1
COMPOUND C1

# FIG. 22

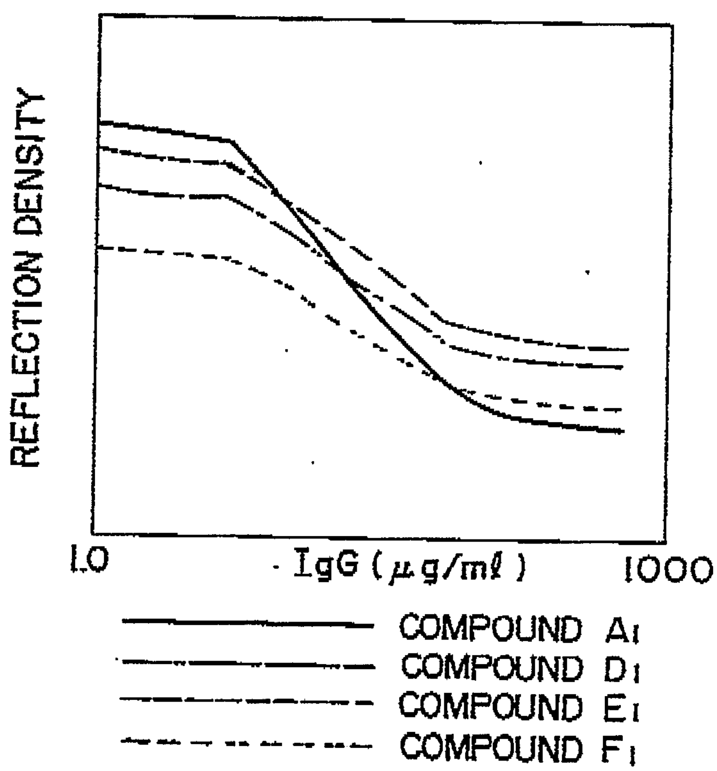
REFLECTION DENSITY
1.0
IgG (μg/mℓ)
1000
COMPOUND A1
COMPOUND D1
COMPOUND E1
COMPOUND F1

# FIG. 23

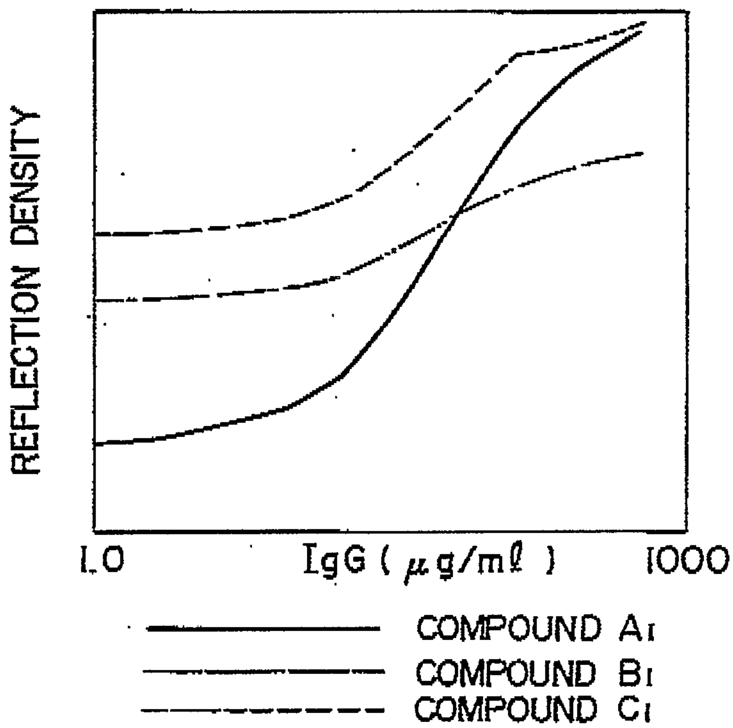
REFLECTION DENSITY
1.0
IgG ( μg/mℓ )
1000
COMPOUND A1
COMPOUND B1
COMPOUND C1

# FIG. 24

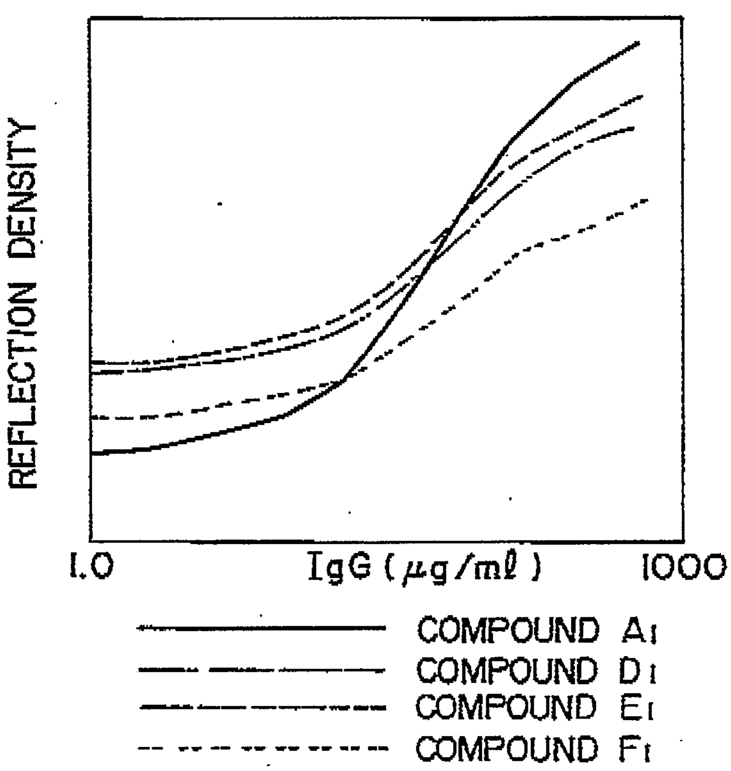
REFLECTION DENSITY
1.0
IgG ( μg/mℓ )
1000
COMPOUND A1
COMPOUND D1
COMPOUND E1
COMPOUND F1

FIG. 25

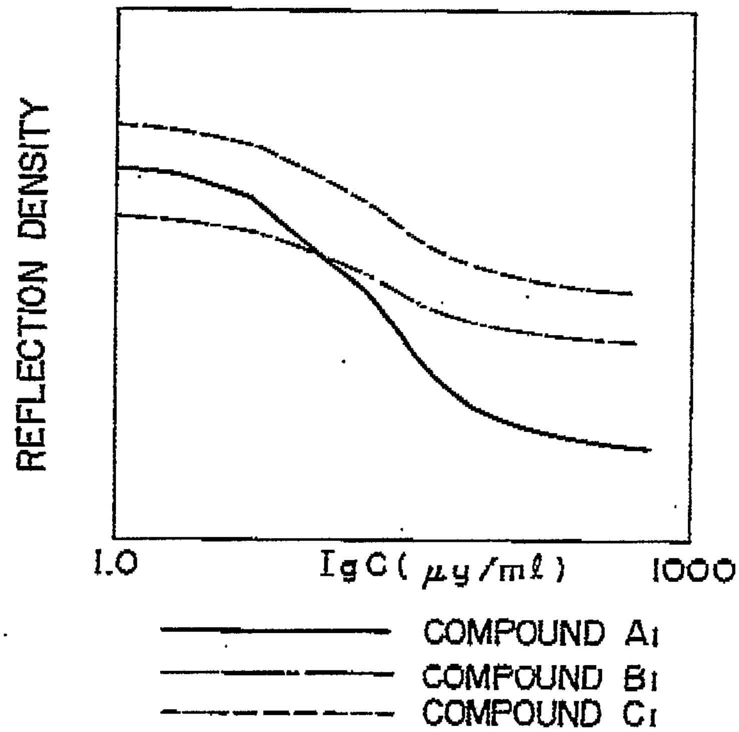
REFLECTION DENSITY
1.0
IgC ( μg/mℓ )
1000
COMPOUND A1
COMPOUND B1
COMPOUND C1

FIG. 26

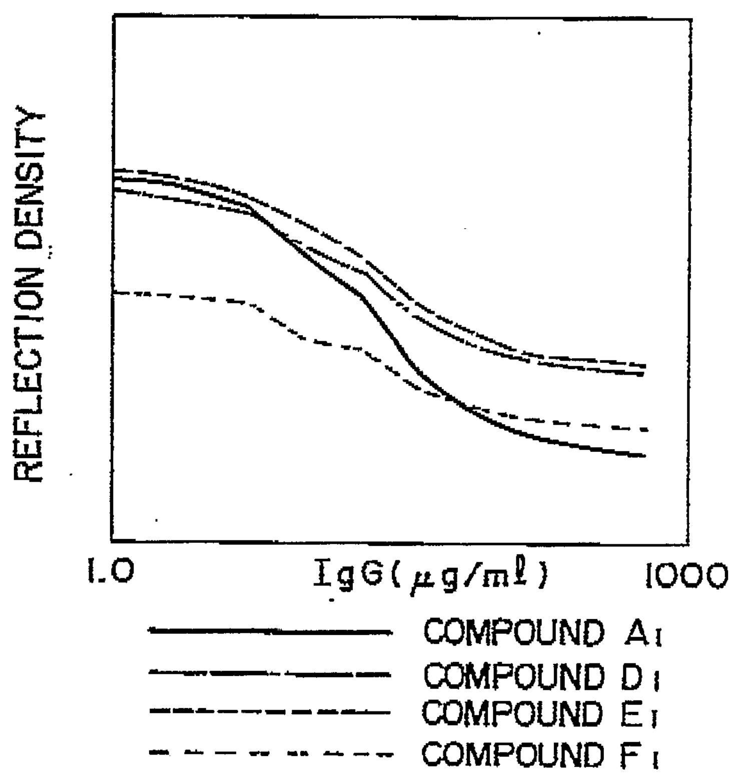
REFLECTION DENSITY
1.0
IgG ( μg/mℓ )
1000
COMPOUND A1
COMPOUND D1
COMPOUND E1
COMPOUND F1

# FIG. 27

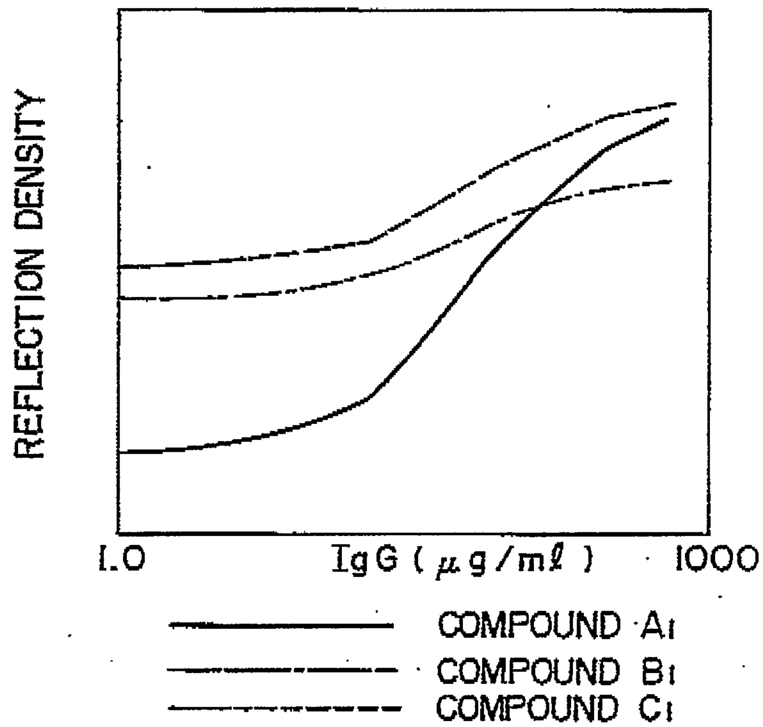
REFLECTION DENSITY
1.0
IgG ( μg / mℓ )
1000
COMPOUND A1
COMPOUND B1
COMPOUND C1

# FIG. 28

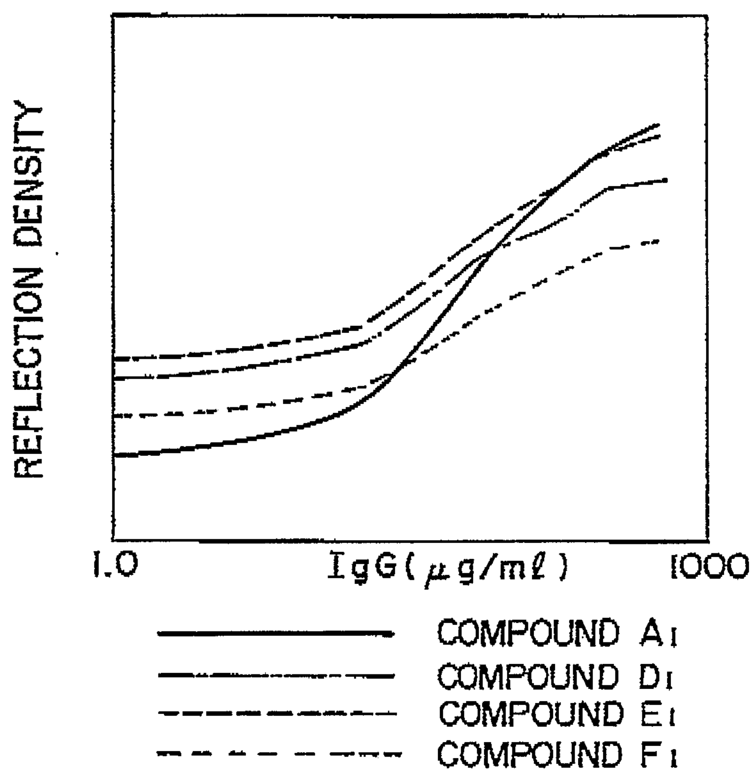
REFLECTION DENSITY
1.0
IgG ( μg / mℓ )
1000
COMPOUND A1
COMPOUND D1
COMPOUND E1
COMPOUND F1

FIG. 29

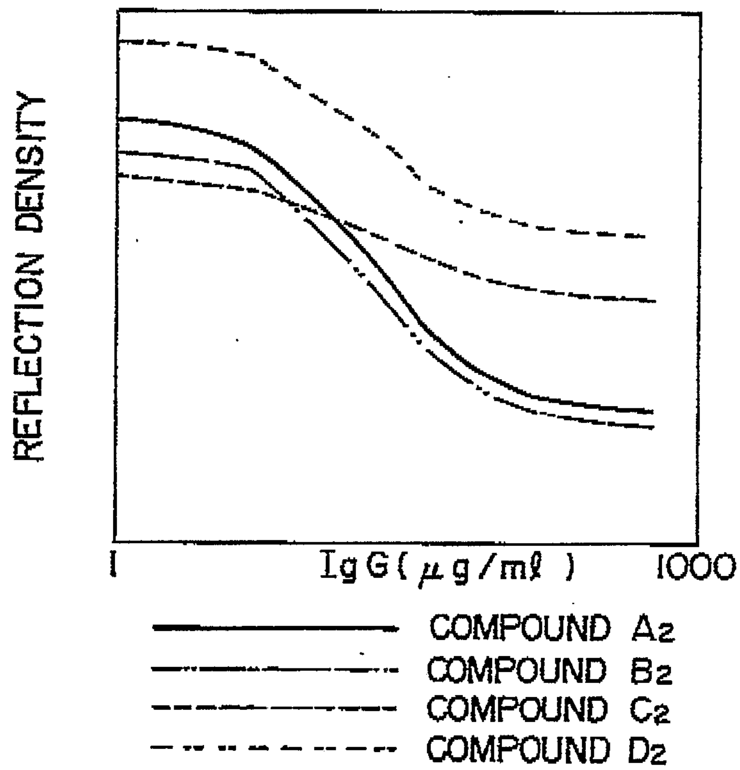
REFLECTION DENSITY
1
IgG (μg/mℓ)
1000
COMPOUND A2
COMPOUND B2
COMPOUND C2
COMPOUND D2

FIG. 30

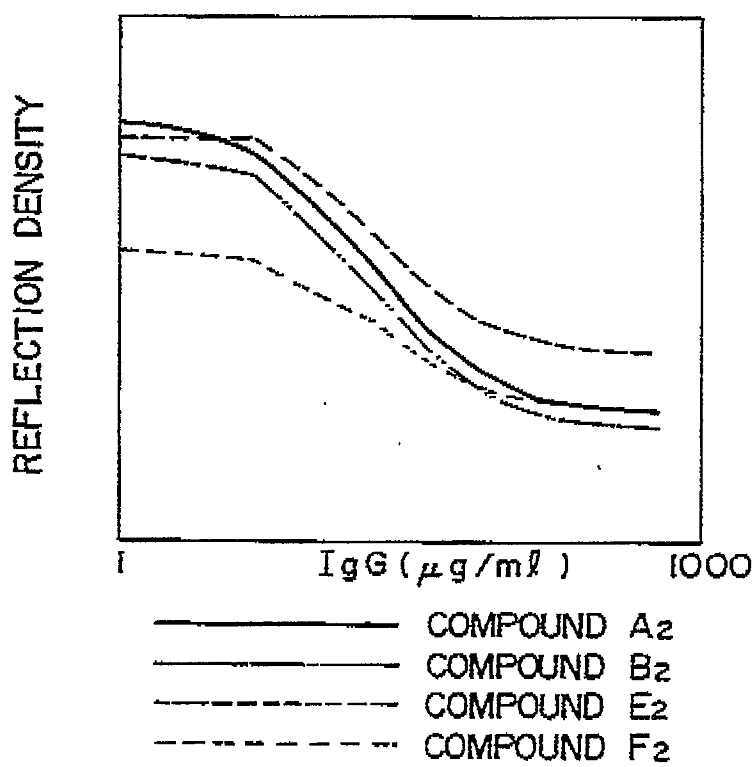
REFLECTION DENSITY
1
IgG (μg/mℓ)
1000
COMPOUND A2
COMPOUND B2
COMPOUND E2
COMPOUND F2

# FIG. 31

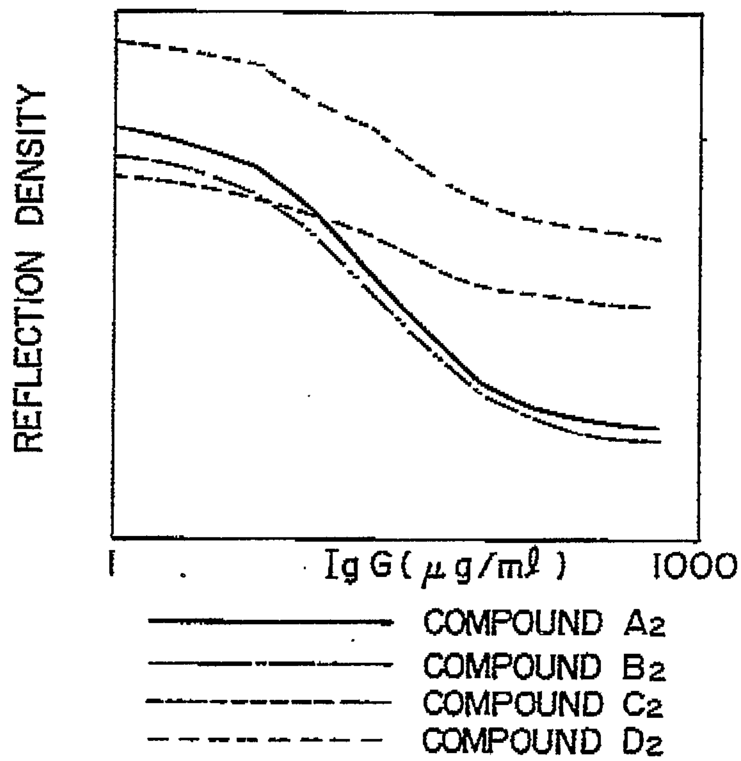
REFLECTION DENSITY
1
IgG(μg/mℓ)
1000
COMPOUND A2
COMPOUND B2
COMPOUND C2
COMPOUND D2

# FIG. 32

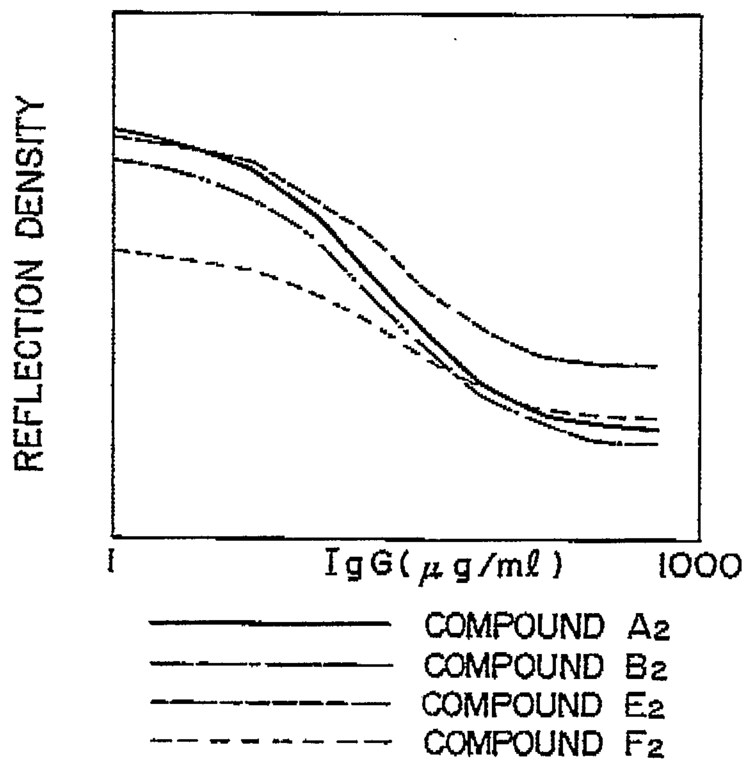
REFLECTION DENSITY
1
IgG(μg/mℓ)
1000
COMPOUND A2
COMPOUND B2
COMPOUND E2
COMPOUND F2

FIG. 33

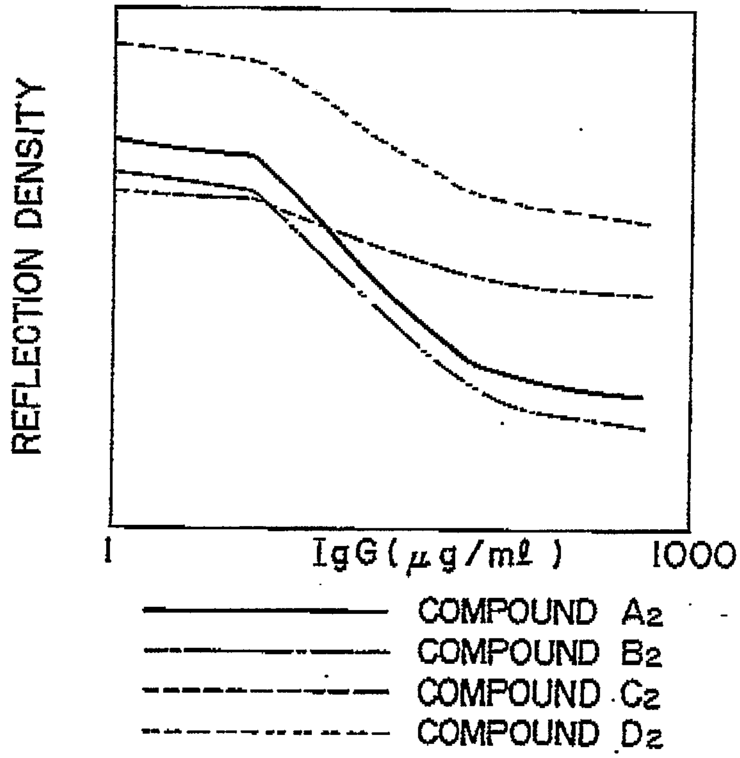

REFLECTION DENSITY
1
IgG (μg/mℓ)
1000
COMPOUND A2
COMPOUND B2
COMPOUND C2
COMPOUND D2


FIG. 34

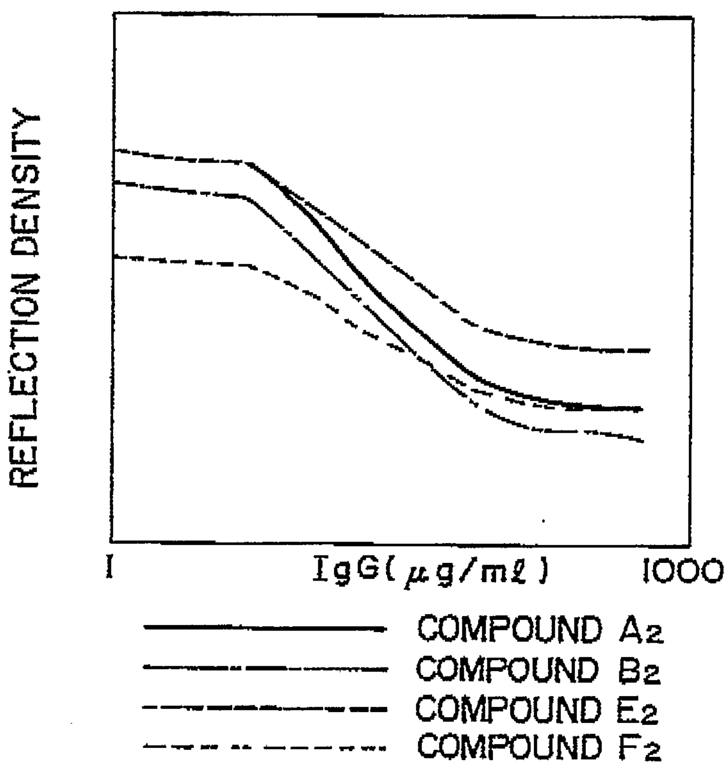

REFLECTION DENSITY
1
IgG (μg/mℓ)
1000
COMPOUND A2
COMPOUND B2
COMPOUND E2
COMPOUND F2

# FIG. 35

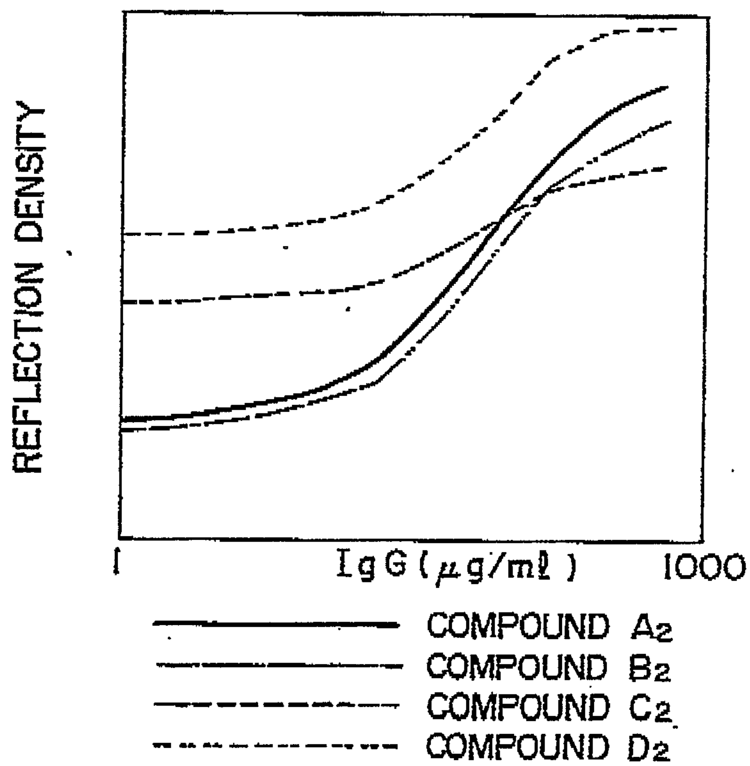
REFLECTION DENSITY
I
IgG (μg/mℓ)
1000
COMPOUND A2
COMPOUND B2
COMPOUND C2
COMPOUND D2

# FIG. 36

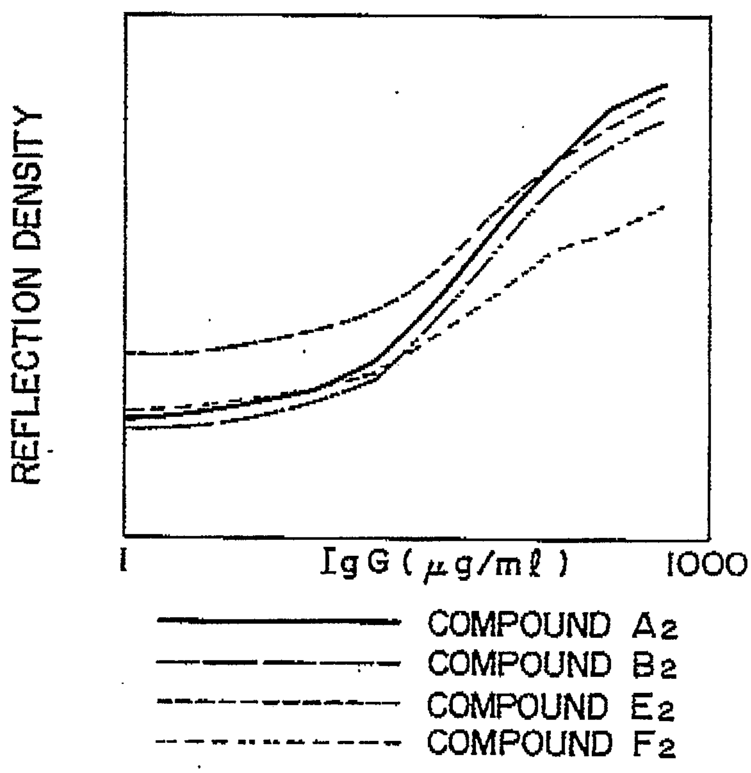
REFLECTION DENSITY
I
IgG (μg/mℓ)
1000
COMPOUND A2
COMPOUND B2
COMPOUND E2
COMPOUND F2

FIG. 37

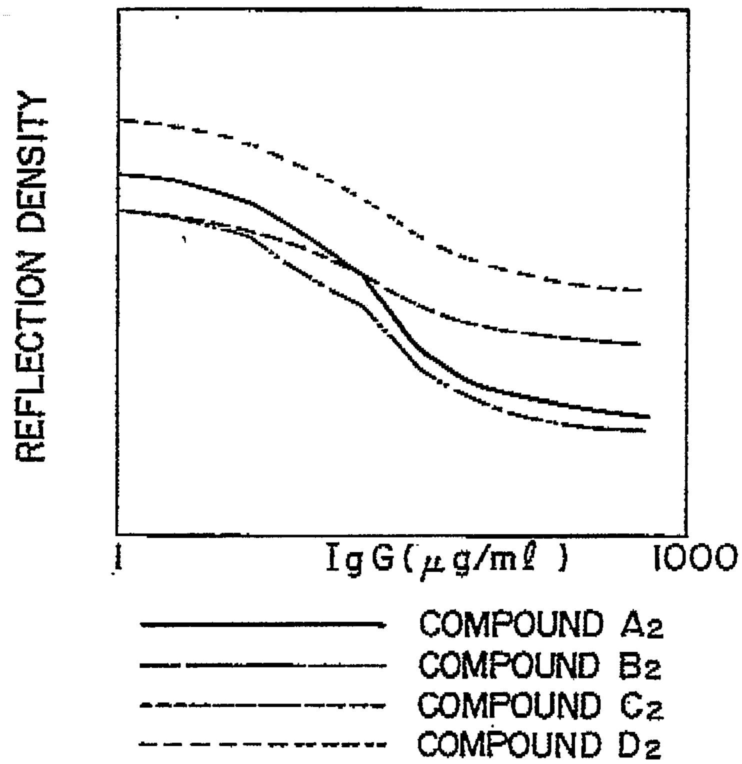
REFLECTION DENSITY
1
IgG(μg/mℓ)
1000
COMPOUND A2
COMPOUND B2
COMPOUND C2
COMPOUND D2

FIG. 38

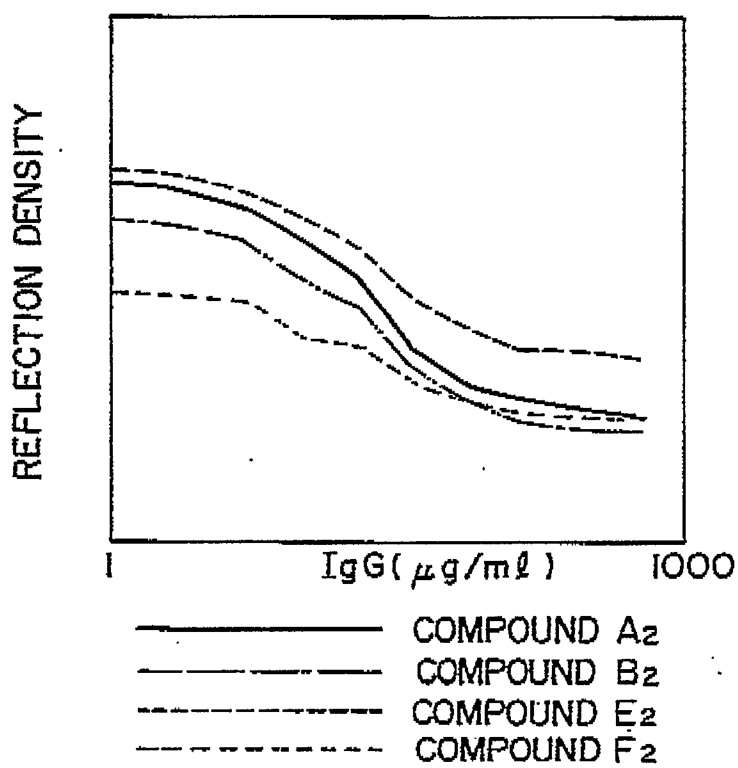
REFLECTION DENSITY
1
IgG(μg/mℓ)
1000
COMPOUND A2
COMPOUND B2
COMPOUND E2
COMPOUND F2

FIG. 39

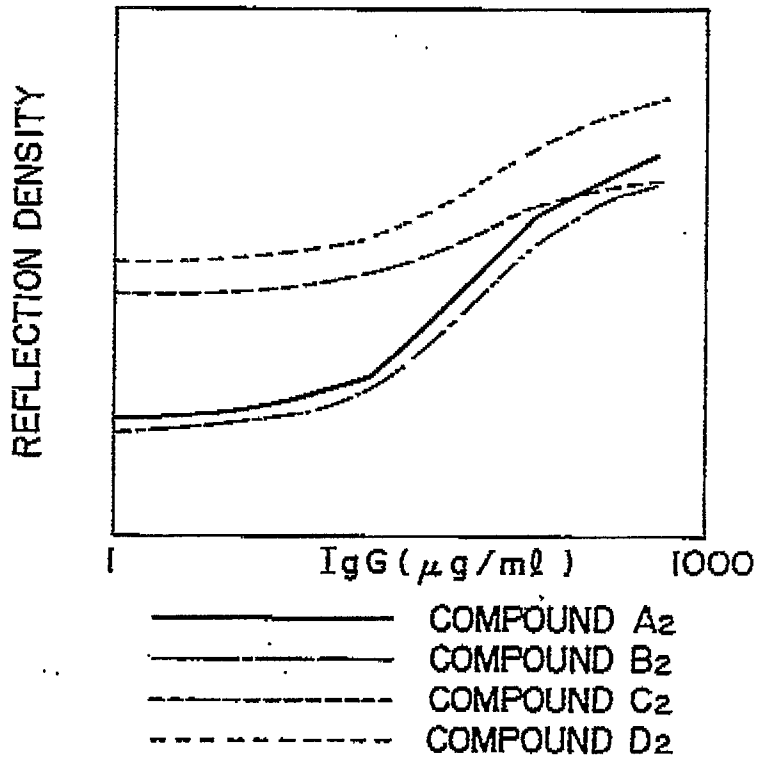
REFLECTION DENSITY
I
IgG (μg/mℓ)
1000
COMPOUND A2
COMPOUND B2
COMPOUND C2
COMPOUND D2

FIG. 40

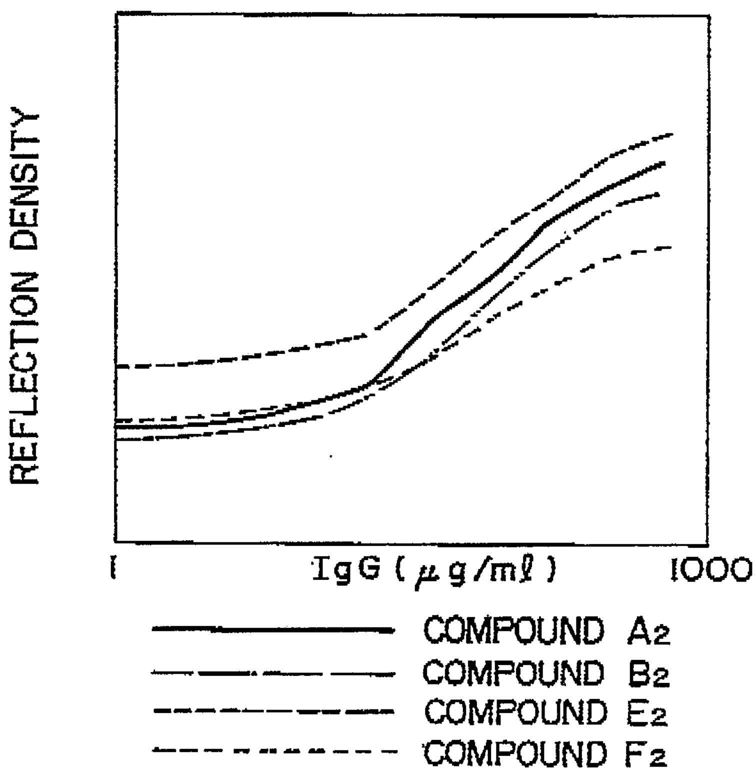
REFLECTION DENSITY
I
IgG (μg/mℓ)
1000
COMPOUND A2
COMPOUND B2
COMPOUND E2
COMPOUND F2

Fig. 41

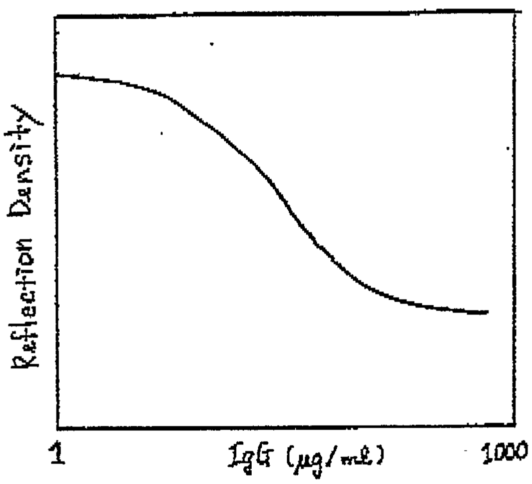


Fig. 42

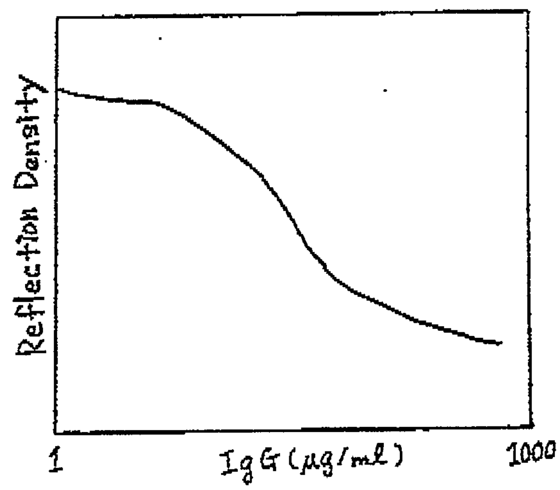


Fig. 43

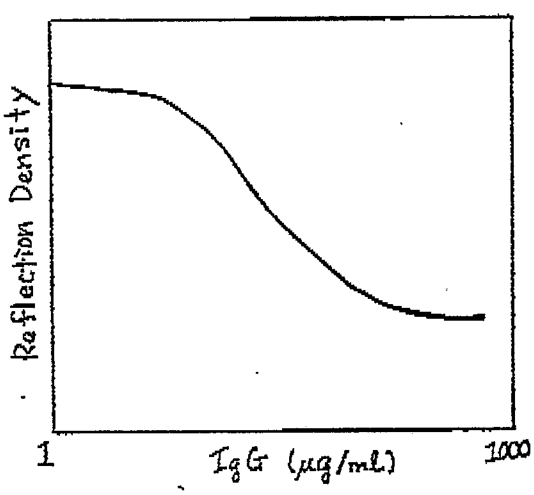


Fig. 44